# EUROPEAN PATENT SPECIFICATION

(11) **EP 4 551 145 B1**
(45) Date of publication and mention of the grant of the patent: **16.09.2026**
(21) Application number: 23744371.8
(22) Date of filing: 07.07.2023
(51) Int. Cl.: A61B 34/10, A61B 34/20

(54) **SYSTEM, AND METHOD FOR DETERMINING AN ALIGNMENT OF A KNEE PROSTHESIS IN A BONE OF A PATIENT**
SYSTEM UND VERFAHREN ZUR BESTIMMUNG EINER AUSRICHTUNG EINER KNIEPROTHESE IN EINEM KNOCHEN EINES PATIENTEN
SYSTÈME ET PROCÉDÉ POUR DÉTERMINER UN ALIGNEMENT D'UNE PROTHÈSE DU GENOU DANS UN OS D'UN PATIENT

(30) Priority: 07.07.2022 US 202263359200 P; 04.11.2022 US 202263422508 P
(43) Date of publication of application: 14.05.2025
(73) Proprietor: DePuy Ireland Unlimited Company, Ringaskiddy, County Cork (IE)
(72) Inventor: DRESSLER, Matthew R., Raynham, Massachusetts 02767-0350 (US); CLARY, Chadd, Lyons, Colorado 80540 (US); FITZPATRICK, Clare K., Lyons, Colorado 80540 (US); RULLKOETTER, Paul J., Lyons, Colorado 80540 (US)
(74) Representative: Murgitroyd & Company
(86) International application number: PCT/EP2023/068851
(87) International publication number: WO 2024/008923

(56) References cited:
- WO-A2-2017/106294
- US-A1- 2010 076 563
- LAMBRECHTS ADRIAAN ET AL: "Artificial Intelligence Based Patient-Specific Preoperative Planning Algorithm for Total Knee Arthroplasty", FRONTIERS IN ROBOTICS AND AI, vol. 9, 8 March 2022 (2022-03-08), XP093082953, DOI: 10.3389/frobt.2022.840282

## Description

### CROSS-REFERENCE TO RELATED APPLICATIONS

This application claims the benefit of U.S. Provisional Patent Application No. 63/359,200, filed July 7, 2022, and of U.S. Provisional Patent Application No. 63/422,508, filed November 4, 2022, both entitled "APPARATUS, SYSTEM, AND METHOD FOR DETERMINING AN ALIGNMENT OF A KNEE PROSTHESIS IN A BONE OF A PATIENT."

### TECHNICAL FIELD

The present disclosure relates generally to computer-assisted orthopaedic systems for use in the planning and performance of orthopaedic procedures and, more particularly, to technologies for determining an alignment of a knee prosthesis for a patient undergoing a knee replacement procedure.

### BACKGROUND

Joint arthroplasty is a well-known surgical procedure by which a diseased and/or damaged natural joint is replaced by a prosthetic joint. For example, a typical knee prosthesis includes a tibial tray, a femoral prosthesis, and a polymer insert or bearing positioned between the tibial tray and the femoral prosthesis. Depending on the severity of the damage to the patient's joint, orthopaedic prostheses of varying mobility may be used. For example, the knee prosthesis may include a "fixed" tibial insert in cases wherein it is desirable to limit the movement of the knee prosthesis, such as when significant soft tissue damage or loss is present. Alternatively, the knee prosthesis may include a "mobile" tibial insert in cases wherein a greater degree of freedom of movement is desired. Additionally, the knee prosthesis may be a total knee prosthesis designed to replace the femoral-tibial interface of both condyles of the patient's femur or a uni-compartmental (or uni-condylar) knee prosthesis designed to replace the femoral-tibial interface of a single condyle of the patient's femur.

The type of orthopedic knee prosthesis used to replace a patient's natural knee may also depend on whether the patient's posterior cruciate ligament is retained or sacrificed (i.e., removed) during surgery. For example, if the patient's posterior cruciate ligament is damaged, diseased, and/or otherwise removed during surgery, a posterior-stabilized knee prosthesis may be used to provide additional support and/or control at later degrees of flexion. Alternatively, if the posterior cruciate ligament is intact, a cruciate-retaining knee prosthesis may be used.

An orthopaedic surgeon may perform some amount of surgical planning to, for example, select a type, size, position, and orientation of a knee prosthesis relative to the patient's boney anatomy. Such planning may be performed manually by the orthopaedic surgeon based on an examination of the patient, pre-operative medical images of the patient's boney anatomy, or intra-operative data gathered in the operating room prior to beginning and/or during the surgical procedure.

WO 2017/106294 describes systems and methods for determining implant configuration or design of implants by taking into account soft tissues and utilizing patient-specific simulations of activities of daily living.

### SUMMARY

According to a first aspect of the present invention, there is provided an orthopaedic surgical planning method, as defined in claim 1. Optional further features of the orthopaedic surgical planning method are defined in the dependent claims. According to the present disclosure, an orthopaedic surgical planning method comprises identifying anatomical parameters of a knee joint of a patient, determining target kinematics of the knee joint based on the patient's pre-implantation anatomy, determining predicted kinematics for a number of implant alignment options for a knee prosthesis when implanted in the knee joint, identifying a recommended implant alignment of the knee prosthesis based on the target kinematics and the predicted kinematics of the knee prosthesis, and preparing a patient-specific surgical plan incorporating the recommended implant alignment, all by a computer system. Determining the predicted kinematics may include the computer system inputting a type and a size of the knee prosthesis and the identified anatomical parameters into a mathematical model representative of the kinematics of the knee prosthesis when implanted and operating the mathematical model to determine the predicted kinematics of the knee prosthesis for each alignment of the number of implant alignment options.

In some embodiments, determining the target kinematics of the knee joint may include determining a target ligament elongation of a ligament of the knee joint over a range of flexion. Operating the mathematical model to determine the predicted kinematics of the patient's knee joint may include determining a predicted ligament elongation of the ligament over the range of flexion. Identifying the recommended implant alignment may include calculating a difference between the target ligament elongation and the predicted ligament elongation over the range of flexion for each alignment of the number of implant alignment options and recommending the alignment corresponding to a least difference between the target ligament elongation and the predicted ligament elongation over the range of flexion.

In some embodiments, calculating the difference between the target ligament elongation and the predicted ligament elongation over the range of flexion may include calculating a sum of absolute differences between the target ligament elongation and the predicted ligament elongation in a number of positions throughout the range of flexion. Recommending the alignment corresponding to the least difference may include recommending the alignment corresponding to the least sum of absolute differences.

In some embodiments, the method may further comprise displaying, by the computer system, a graph of ligament elongations for the recommended alignment over the range of flexion. The graph may include indicia of the target ligament elongation over the range of flexion and indicia of the predicted ligament elongation over the range of flexion. The patient-specific surgical plan may include the graph of ligament elongations.

In some embodiments, the ligament may be at least one of a medial collateral ligament, a lateral collateral ligament, a posterior cruciate ligament, an anterolateral ligament, and a posterior capsule of the knee joint of the patient.

In some embodiments, determining the target ligament elongation of the ligament of the knee joint over the range of flexion may include determining the target ligament elongation of each of the medial collateral ligament, the lateral collateral ligament, and the posterior cruciate ligament of the knee joint. Determining the predicted ligament elongation of the ligament over the range of flexion may include determining the predicted ligament elongation of each of the medial collateral ligament, the lateral collateral ligament, and the posterior cruciate ligament. Calculating the difference between the target ligament elongation and the predicted ligament elongation may include calculating an absolute difference between the target ligament elongation and the predicted ligament elongation of each of the medial collateral ligament, the lateral collateral ligament, and the posterior cruciate ligament, and calculating the sum of the absolute differences for the medial collateral ligament, the lateral collateral ligament, and the posterior cruciate ligament. The method may include recommending the alignment having the least sum of the absolute differences.

In some embodiments, determining the target ligament elongation of the ligament of the knee joint over the range of flexion may further include determining the target ligament elongation of each of the anterolateral ligament and the posterior capsule of the knee joint. Determining the predicted ligament elongation of the ligament over the range of flexion may further include determining the predicted ligament elongation of each of the anterolateral ligament and the posterior capsule of the knee joint. Calculating the difference between the target ligament elongation and the predicted ligament elongation may further include calculating an absolute difference between the target ligament elongation and the predicted ligament elongation of each of the anterolateral ligament and the posterior capsule of the knee joint. Calculating the sum of the absolute differences may include calculating the sum of the absolute differences for the medial collateral ligament, the lateral collateral ligament, the posterior cruciate ligament, the anterolateral ligament, and the posterior capsule of the knee joint.

In some embodiments, the patient-specific surgical plan may include a number of planned resections of the patient's femur and at least one planned resection of the patient's tibia to prepare the patient's bone to receive the knee prosthesis.

In some embodiments, identifying anatomical parameters of the knee joint may include identifying anatomical parameters of the knee joint of the patient based on a set of medical images of the patient's pre-operative anatomy. The method may comprise acquiring the set of medical images through at least one of a CT scan, X-Ray imaging, and ultrasound imaging of the patient's pre-operative anatomy.

In some examples of a method which is useful as background to the disclosure, the method may comprise performing, with the computer system, an intra-operative bone registration procedure on the knee joint of the patient to identify a number of landmarks on the patient's pre-implantation anatomy, wherein the anatomical parameters include the number of landmarks. The example method may further comprise tracking, with the computer system, motion of the knee joint over a range of flexion based on the identified landmarks, wherein determining the target kinematics of the knee joint includes determining the target kinematics based on the motion of the knee joint. The example method may further comprise inserting a joint distractor instrument in the knee joint, and operating the joint distractor instrument to adjust a distance between the patient's femur and tibia over the range of flexion.

In some embodiments, determining target kinematics of the knee joint based on the patient's pre-implantation anatomy may include determining a first target kinematics of the knee joint based on a set of medical images of the patient's pre-operative anatomy. Determining target kinematics of the knee joint based on the patient's pre-implantation anatomy may further include determining a second target kinematics of the knee joint based on an intra-operative bone registration procedure on the knee joint of the patient. Determining predicted kinematics for a number of implant alignment options for a knee prosthesis when implanted in the knee joint may include operating the mathematical model, after inputting a type and a size of the knee prosthesis and anatomical parameters identified from the set of medical images, to determine first predicted kinematics of the knee prosthesis for each alignment of the number of implant alignment option. Determining predicted kinematics for a number of implant alignment options for a knee prosthesis when implanted in the knee joint may further include operating the mathematical model, after inputting the type and the size of the knee prosthesis and intra-operatively identified anatomical parameters, to determine second predicted kinematics of the knee prosthesis for each alignment of the number of implant alignment option. Identifying a recommended implant alignment of the knee prosthesis may include identifying a first possible alignment based on the first target kinematics and the first predicted kinematics of the knee prosthesis. Identifying a recommended implant alignment of the knee prosthesis may further include a second possible alignment based on the second target kinematics and the second predicted kinematics of the knee prosthesis. The method may further comprise displaying indicia of at least one of the first possible alignment and the second possible alignment, resecting the patient's bones to prepare the bones to receive the knee prosthesis in one of the first possible alignment and the second possible alignment, and/or implanting the knee prosthesis.

In some embodiments, the method may further comprise determining the implanted kinematics of the knee prosthesis over a range of flexion and displaying an indicia of the implanted kinematics and at least one of the first target kinematics, the second target kinematics, the first predicted kinematics, and the second predicted kinematics.

In some embodiments, determining the predicted kinematics of the knee prosthesis may include inputting a number of sizes of knee prosthesis and the identified anatomical parameters into the mathematical model, each of size of knee prosthesis being of the same type, and operating the mathematical model to determine the predicted kinematics for each size of knee prosthesis at each alignment of the number of implant alignment options.

In some embodiments, determining the target kinematics may include inputting the anatomical parameters into a statistical shape-function model and operating the statistical shape-function model to generate the target kinematics of the knee joint.

In some embodiments, the mathematical model may be a linear response model configured to receive anatomic parameters, knee prosthesis size, and knee prosthesis type as inputs and to generate predicted kinematics of a knee prosthesis as an output.

In some embodiments, the linear response model may be further configured to generate predicted mechanics of a knee prosthesis as an output. The mechanics may include at least one of predicted contact forces between components of the knee prosthesis and predicted contact forces between components of the knee prosthesis and the patient's bones.

According to another aspect, an orthopaedic surgical planning system comprises a computer system configured to automatically identify anatomical parameters of the knee joint, determine target kinematics of the knee joint based on the patient's pre-implantation anatomy, determine predicted kinematics for a number of implant alignment options for a knee prosthesis when implanted in the knee joint, identify a recommended alignment of the knee prosthesis based on the target kinematics and the predicted kinematics of the knee prosthesis, and prepare a patient-specific surgical plan including the recommended implant alignment. To determine the predicted kinematics, the computer system may be configured to automatically input a type and a size of the knee prosthesis and the identified anatomical parameters into a mathematical model representative of the kinematics of the knee prosthesis when implanted and operate the mathematical model to determine the predicted kinematics of the knee prosthesis for each alignment of the number of implant alignment options.

In some embodiments, the orthopaedic surgical planning system further comprises a robotic system including a cutting tool configured to resect the patient's bones according to the patient-specific surgical plan.

In some embodiments, the orthopaedic surgical planning system further comprises a bone registration tool for identifying a number of landmarks on the patient's pre-implantation anatomy with the computer system. The anatomical parameters may include the number of landmarks. The computer system may be configured to determine target kinematics of the knee joint by tracking motion of the knee joint based on the identified landmarks to determine target kinematics of the knee joint over a range of flexion.

In some embodiments, the computer system may include a display operable to display a graph of ligament elongations for the recommended alignment over a range of flexion. The graph may include indicia of target ligament elongation over the range of flexion and indicia of predicted ligament elongation over the range of flexion.

In some embodiments, the mathematical model may be a linear response model configured to receive anatomic parameters, knee prosthesis size, and knee prosthesis type as inputs and generate predicted kinematics of a knee prosthesis as an output. In some embodiments, the linear response model may be further configured to generate predicted mechanics of a knee prosthesis as an output. The mechanics may include at least one of predicted contact forces between components of the knee prosthesis and predicted contact forces between components of the knee prosthesis and the patient's bones.

According to yet another aspect, an orthopaedic surgical planning method comprises identifying anatomical parameters of a knee joint of a patient, determining target performance of the knee joint based on the patient's pre-implantation anatomy, determining predicted performance for a number of implant alignment options for a knee prosthesis when implanted in the knee joint, identifying a recommended implant alignment of the knee prosthesis based on the target performance and the predicted performance of the knee prosthesis, and preparing a patient-specific surgical plan incorporating the recommended implant alignment, all by a computer system. Determining the predicted performance may include the computer system inputting a type and a size of the knee prosthesis and the identified anatomical parameters into a mathematical model representative of the kinematics and mechanics of the knee prosthesis when implanted and operating the mathematical model to determine the predicted performance of the knee prosthesis for each alignment of the number of implant alignment options.

In some embodiments, the predicted performance may include the predicted kinematics for the number of implant alignment options for the knee prosthesis when implanted in the knee joint.

In some embodiments, determining the target performance of the knee joint may include determining a target ligament elongation of a ligament of the knee joint over a range of flexion. Operating the mathematical model to determine the predicted performance of the patient's knee joint may include determining a predicted ligament elongation of the ligament over the range of flexion. Identifying the recommended implant alignment may include calculating a difference between the target ligament elongation and the predicted ligament elongation over the range of flexion for each alignment of the number of implant alignment options and recommending the alignment corresponding to a least difference between the target ligament elongation and the predicted ligament elongation over the range of flexion.

In some embodiments, calculating the difference between the target ligament elongation and the predicted ligament elongation over the range of flexion may include calculating a sum of absolute differences between the target ligament elongation and the predicted ligament elongation in a number of positions throughout the range of flexion. Recommending the alignment corresponding to the least difference may include recommending the alignment corresponding to the least sum of absolute differences.

In some embodiments, the predicted performance may include the predicted mechanics for the number of implant alignment options for the knee prosthesis when implanted in the knee joint.

In some embodiments, identifying the recommended implant alignment of the knee prosthesis based on the target performance and the predicted performance of the knee prosthesis may include selecting the implant alignment based on one of predicted contact forces between components of the knee prosthesis and predicted contact forces between components of the knee prosthesis and the patient's bones.

According to still another aspect, an orthopaedic surgical planning method comprises identifying anatomical parameters of a knee joint of a patient, determining a subset of implant alignment options for a knee prosthesis when implanted in the knee joint from among a larger set of implant alignment options, where the subset of implant alignment options consists of each implant alignment from the larger set of implant alignment options that satisfies one or more flexion-extension gap criteria, determining a target ligament elongation of at least one ligament of the knee joint over a range of flexion based on the patient's pre-implantation anatomy, determining for each implant alignment of the subset of implant alignment options, a predicted ligament elongation of the at least one ligament over the range of flexion, identifying at least one recommended implant alignment of the knee prosthesis based on the target ligament elongation and the predicted ligament elongations, and incorporating the at least one recommended implant alignment into a patient-specific surgical plan, all by a computer system.

In some embodiments, the computer system does not determine predicted ligament elongations for each implant alignment of the larger set of implant alignment options other than the subset of implant alignment options.

In some embodiments, determining the subset of implant alignment options that satisfy the one or more flexion-extension gap criteria may include, for each implant alignment of the larger set of implant alignment options, virtually positioning a femoral prosthesis of the knee prosthesis relative to a femur of the knee joint according to the implant alignment, determining a predicted gap between the virtually positioned femoral prosthesis and a planned tibial resection plane according to the implant alignment, and comparing the predicted gap to a reference value.

In some embodiments, determining the predicted gap for each implant alignment may include finding a smallest distance between the virtually positioned femoral prosthesis and the planned tibial resection plane. Determining the predicted gap for each implant alignment may include calculating a distance between a predetermined point along the sagittal curvature of the virtually positioned femoral prosthesis and the planned tibial resection plane.

Determining the subset of implant alignment options that satisfy the one or more flexion-extension gap criteria may further include generating a performance metric for each implant alignment of the larger set of implant alignment options by calculating the predicted gap between the virtually positioned femoral prosthesis and the planned tibial resection plane at each of a plurality of flexion-extension angles, determining an absolute difference between the predicted gap at each of the plurality of flexion-extension angles and a corresponding reference value, and summing the absolute differences. Generating the performance metric may further include combining a medial-side performance metric and a lateral-side performance metric.

In some embodiments, identifying the at least one recommended implant alignment may include calculating a difference between the target ligament elongation and the predicted ligament elongation for each implant alignment of the subset of implant alignment options and recommending an implant alignment corresponding to a least difference between the target ligament elongation and the predicted ligament elongation over the range of flexion. Calculating the difference between the target ligament elongation and the predicted ligament elongation over the range of flexion may include calculating a sum of absolute differences between the target ligament elongation and the predicted ligament elongation in a number of poses throughout the range of flexion. Recommending the implant alignment corresponding to the least difference may include recommending an implant alignment corresponding to the least sum of absolute differences.

In some embodiments, the at least one ligament may be at least one of a medial collateral ligament, a lateral collateral ligament, a posterior cruciate ligament, an anterolateral ligament, and a posterior capsule of the knee joint of the patient. Determining the target ligament elongation of the at least one ligament of the knee joint over the range of flexion may include determining the target ligament elongation of each of the medial collateral ligament, the lateral collateral ligament, and the posterior cruciate ligament of the knee joint. Determining the predicted ligament elongation of the at least one ligament over the range of flexion may include determining the predicted ligament elongation of each of the medial collateral ligament, the lateral collateral ligament, and the posterior cruciate ligament. Calculating the difference between the target ligament elongation and the predicted ligament elongation may include calculating an absolute difference between the target ligament elongation and the predicted ligament elongation of each of the medial collateral ligament, the lateral collateral ligament, and the posterior cruciate ligament, and calculating the sum of the absolute differences for the medial collateral ligament, the lateral collateral ligament, and the posterior cruciate ligament. Recommending the implant alignment may include recommending an implant alignment having the least sum of the absolute differences.

In some embodiments, identifying anatomical parameters of the knee joint may include identifying anatomical parameters of the knee joint of the patient based on a set of medical images of the patient's pre-operative anatomy. The method may comprise acquiring the set of medical images through at least one of a CT scan, X-Ray imaging, and ultrasound imaging of the patient's pre-operative anatomy.

In some embodiments, the method may further comprise performing, with the computer system, an intra-operative bone registration procedure on the knee joint of the patient to identify a number of landmarks on the patient's pre-implantation anatomy, where the anatomical parameters include the number of landmarks. The method may further comprise tracking, with the computer system, motion of the knee joint over a range of flexion based on the identified landmarks, where determining the target kinematics of the knee joint includes determining the target kinematics based on the motion of the knee joint. In some embodiments, the method may further comprise inserting a joint distractor instrument in the knee joint and operating the joint distractor instrument to adjust a distance between the patient's femur and tibia over the range of flexion.

In some embodiments, determining, for each implant alignment of the subset of implant alignment options, the predicted ligament elongation of the at least one ligament over the range of flexion may include inputting anatomic parameters, knee prosthesis size, knee prosthesis type, and the implant alignment into a linear response model and operating the linear response model to generate the predicted ligament elongation.

According to yet another aspect, an orthopaedic surgical planning system may comprise a computer system configured to automatically identify anatomical parameters of a knee joint of a patient, determine a subset of implant alignment options for a knee prosthesis when implanted in the knee joint from among a larger set of implant alignment options, where the subset of implant alignment options consists of each implant alignment from the larger set of implant alignment options that satisfies one or more flexion-extension gap criteria, determine a target ligament elongation of at least one ligament of the knee joint over a range of flexion based on the patient's pre-implantation anatomy, determine, for each implant alignment of the subset of implant alignment options, a predicted ligament elongation of the at least one ligament over the range of flexion, identify at least one recommended implant alignment of the knee prosthesis based on the target ligament elongation and the predicted ligament elongations, and incorporate the at least one recommended implant alignment into a patient-specific surgical plan.

In some embodiments, the orthopaedic surgical planning system may further comprise a bone registration tool for identifying a number of landmarks on the patient's pre-implantation anatomy with the computer system. The anatomical parameters may include the number of landmarks. In some embodiments, the orthopaedic surgical planning system may further comprise a robotic system including a cutting tool configured to resect one or more of the patient's bones according to the patient-specific surgical plan.

According to still another aspect, an orthopaedic surgical planning method may comprise identifying anatomical parameters of a knee joint of a patient, determining target kinematics of the knee joint based on the patient's pre-implantation anatomy, determining one or more predicted gaps between a femoral prosthesis and a tibial prosthesis when implanted in the knee joint at each of a first set of implant alignment options, determining a second set of implant alignment options from among the first set of implant alignment options based at least in part on the one or more predicted gaps, determining predicted kinematics for each implant alignment of the second set of implant alignment options, identifying at least one recommended implant alignment of the femoral and tibial prostheses based on the target kinematics and the predicted kinematics, and incorporating the at least one recommended implant alignment into a patient-specific surgical plan, all by a computer system. Determining the predicted kinematics may include the computer system inputting the identified anatomical parameters into a mathematical model representative of the kinematics of the femoral and tibial prostheses when implanted and operating the mathematical model to determine the predicted kinematics of the femoral and tibial prostheses for each implant alignment of the second set of implant alignment options.

The first set of implant alignment options may reflect a first set of degrees of freedom for positioning of the femoral and tibial prostheses relative to bones of the knee joint, the second set of implant alignment options may reflect a second set of degrees of freedom for positioning of the femoral and tibial prostheses relative to the bones of the knee joint, and at least one degree of freedom of the second set of degrees of freedom may be constrained relative to the first set of degrees of freedom. In some embodiments, multiple degrees of freedom of the second set of degrees of freedom may be constrained relative to the first set of degrees of freedom. In some embodiments, at least one degree of freedom of the first set of degrees of freedom may be fixed for the second set of implant alignment options.

In some embodiments, the computer system may determine the one or more predicted gaps using the target kinematics and known geometries of the femoral and tibial prostheses. Determining the one or more predicted gaps may include determining, for each implant alignment of the first set of implant alignment options, the predicted gap between the femoral and tibial prostheses at each of a plurality of flexion-extension angles.

In some embodiments, determining the one or more predicted gaps may include determining, for each implant alignment of the first set of implant alignment options, a medial-side predicted gap between the femoral and tibial prostheses and a lateral-side predicted gap between the femoral and tibial prostheses. Determining the one or more predicted gaps may include determining, for each implant alignment of the first set of implant alignment options, the medial-side predicted gap and the lateral-side predicted gap at each of a plurality of flexion-extension angles.

In some embodiments, determining the second set of implant alignment options from among the first set of implant alignment options may include selecting implant alignments for which the one or more predicted gaps are within a target range.

In some embodiments, determining the target kinematics of the knee joint may include determining a target ligament elongation of at least one ligament of the knee joint over a range of flexion. Operating the mathematical model to determine the predicted kinematics of the femoral and tibial prostheses may include determining a predicted ligament elongation of the at least one ligament over the range of flexion. Identifying the at least one recommended implant alignment may include calculating a difference between the target ligament elongation and the predicted ligament elongation over the range of flexion for each implant alignment of the second set of implant alignment options and recommending the implant alignment corresponding to a least difference between the target ligament elongation and the predicted ligament elongation over the range of flexion.

In some embodiments, calculating the difference between the target ligament elongation and the predicted ligament elongation over the range of flexion may include calculating a sum of absolute differences between the target ligament elongation and the predicted ligament elongation in a number of poses throughout the range of flexion. Recommending the implant alignment corresponding to the least difference may include recommending the implant alignment corresponding to the least sum of absolute differences.

In some embodiments, the at least one ligament may be at least one of a medial collateral ligament, a lateral collateral ligament, a posterior cruciate ligament, an anterolateral ligament, and a posterior capsule of the knee joint of the patient.

In some embodiments, the mathematical model may be a linear response model configured to receive anatomic parameters, prostheses size, prostheses type, and implant alignment as inputs and to generate predicted kinematics of the femoral and tibial prostheses as an output. The linear response model may be further configured to generate predicted mechanics of the femoral and tibial prostheses as an output, the mechanics including at least one of (i) predicted contact forces between the femoral prosthesis and the tibial prosthesis and (ii) predicted contact forces between the femoral and tibial prostheses and the bones of the knee joint.

In some embodiments, the method may include adding to the patient-specific surgical plan a number of planned resections of the patient's femur and at least one planned resection of the patient's tibia to prepare the patient's bones to receive the femoral and tibial prostheses at the recommended implant alignment.

According to yet another aspect, an orthopaedic surgical planning system may comprise a computer system configured to perform any of the methods described in the present disclosure.

According to still another aspect, one or more computer-readable media may store a plurality of instructions that, when executed by one or more processors of a computer system, cause the one or more processors to perform any of the methods described in the present disclosure.

### BRIEF DESCRIPTION OF THE DRAWINGS

The detailed description particularly refers to the following figures, in which:
FIG. 1 is a perspective view of an embodiment of an orthopaedic knee prosthesis installed in a patient's surgically-prepared knee joint;
FIG. 2A is a diagram of an embodiment of an orthopedic knee prosthesis system that includes two different types of femoral prostheses, four different types of tibial inserts, and two different types of tibial trays;
FIG. 2B is a side elevation view of an embodiment of a femoral prosthesis of the orthopaedic knee prosthesis of FIG. 1;
FIG. 3 is a block diagram of an embodiment of a computer system for determining positioning of an orthopaedic knee prosthesis;
FIG. 4 is a block diagram of an embodiment of a knee surgical planning and analysis device of the system of FIG. 3;
FIG. 5 is a block diagram of an embodiment of a robotic surgical device of the system of FIG. 3;
FIG. 6 is a flow chart diagram of an embodiment of a method for developing a patient-specific surgical plan for a knee replacement procedure;
FIG. 7 is a flow chart diagram of an embodiment of a process to determine predicted performance of a knee prosthesis across a number of implant alignment options;
FIG. 8 is an embodiment of a visual representation of a patient-specific surgical plan;
FIG. 9 illustrates an embodiment of a heat map representing an output of a cost function calculated during the process of FIG. 7;
FIG. 10 illustrates another embodiment of a heat map representing the output of the cost function calculated during the process of FIG. 7;
FIG. 11 illustrates an embodiment of a user interface depicting the relative laxity of collateral ligaments at various degrees of flexion of the patient's knee joint;
FIG. 12 is a flow chart diagram of another embodiment of a method for developing a patient-specific surgical plan for a knee replacement procedure;
FIG. 13 is a flow chart diagram of an embodiment of a method for developing and executing a patient-specific surgical plan for a knee replacement procedure using medical imaging;
FIG. 14 is a flow chart diagram of an embodiment of a method for developing and executing a patient-specific surgical plan for a knee replacement procedure using intra-operative joint registration; and
FIG. 15 is a flow chart diagram of an embodiment of a method for developing and executing a patient-specific surgical plan for a knee replacement procedure using medical imaging and intra-operatively joint registration.

### DETAILED DESCRIPTION

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the scope of the invention as defined by the appended claims.

Terms representing anatomical references, such as anterior, posterior, medial, lateral, superior, inferior, etcetera, may be used throughout the specification in reference to the orthopaedic implants and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

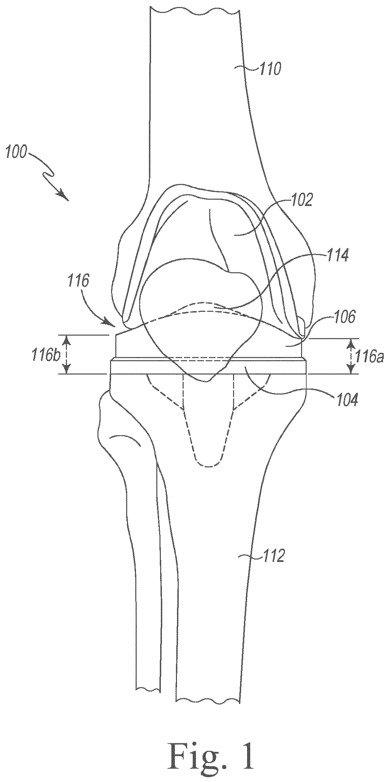

Referring now to FIG. 1, an orthopaedic knee prosthesis 100 includes a femoral prosthesis 102, a tibial tray 104, and a tibial insert 106. The femoral prosthesis 102 is configured to be secured to a surgically-prepared distal end of a patient's femur 110. Similarly, the tibial tray 104 is configured to be secured to a surgically-prepared proximal end of a patient's tibia 112.

The tibial insert 106 is configured to be coupled to the tibial tray 104 and provide an articulation surface for the femoral prosthesis 102 during flexion of the patient's knee joint. For example, the femoral prosthesis 102 includes medial and lateral condyles, which are shaped and configured to engage corresponding medial and lateral articulation surfaces of the tibial insert 106. (A portion of the femoral prosthesis 102 also engages the patient's patella 114 during flexion.) The articulation surfaces of the tibial insert 106 may be symmetrical or asymmetrical. In some embodiments, the asymmetrical tibial inserts may be configured to provide more constraint to the medial condyle of the femoral prosthesis 102 and less constraint to the lateral condyle, thereby permitting additional rotation of the lateral condyle about the more constrained medial condyle.

When the femoral prosthesis 102 is positioned on the surgically-prepared distal end of the patient's femur 110, the femoral prosthesis 102 is spaced from the surgically-prepared proximal end of the patient's tibia 112 by a gap 116 (in which the tibial tray 104 and tibial insert 106 are positioned). The gap 116 may be defined with reference to a medial-side gap 116a at the smallest distance between the surgically-prepared tibia 112 and the medial condyle of the femoral prosthesis 102 and to a lateral-side gap 116b at the smallest distance between the surgically-prepared tibia 112 and the lateral condyle of the femoral prosthesis 102. It will be appreciated that the gap 116 may alternatively be defined in different ways, such as with reference to one or more predetermined points on the femoral prosthesis 102 (e.g., one or more predetermined points along the sagittal curvature of the femoral prosthesis 102, even if other points on the femoral prosthesis 102 may be closer to tibia 112) and/or with reference to the tibial tray 104 or tibial insert 106 (rather than the surgically-prepared tibia 112 itself). In many cases, the size of the gap 116 (including the size(s) of the medial-side gap 116a and the lateral-side gap 116b) will vary as the patient's femur 110 and tibia 112 are moved throughout a range of motion including flexion and extension. Since the sizes of the gaps 116, 116a, 116b are often measured at various flexion-extension angles, the gaps 116, 116a, 116b are frequently referred to as "flexion-extension gaps."

The femoral prosthesis 102 and the tibial tray 104 are formed from a metallic material such as cobalt-chromium or titanium but may be formed from other materials, such as a ceramic material, a polymer material, a bio-engineered material, or the like, in other embodiments. The tibial tray 106 is typically formed from a polymer material such as an ultra-high molecular weight polyethylene (UHMWPE), but may be formed from other materials, such as a ceramic material, a metallic material, a bio-engineered material, or the like, in other embodiments. It should also be appreciated that the tibial tray and tibial insert may be provided as a single, integral tibial prosthesis.

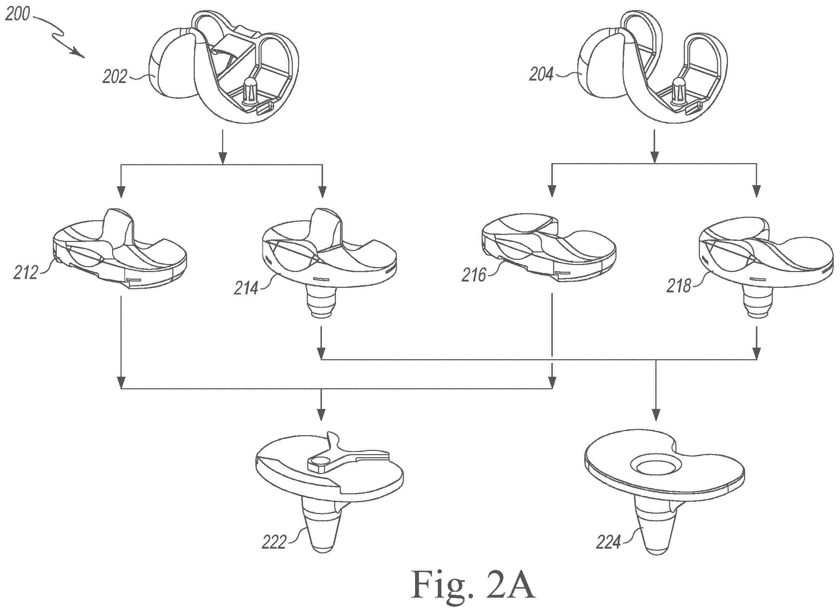

Different types of orthopaedic knee prostheses may be used for different patients based on various factors, including the health of the patient's knee joint. For example, as shown in FIG. 2A, an illustrative orthopedic knee prosthesis system 200 includes two different types of femoral prostheses 202, 204, four different types of tibial inserts 212, 214, 216, 218, and two different types of tibial trays 222, 224. The femoral prosthesis 202 is embodied as a posterior-stabilized (PS) femoral prosthesis and includes a posterior cam. The femoral prosthesis 202 is configured to articular with either the fixed bearing tibial insert 212 or the rotating platform tibial insert 214, each of which includes a post configured to contact the posterior cam of the femoral prosthesis 202. The tibial insert 212 is configured to couple to the tibial tray 222, which is a fixed or non-rotating tibial tray. That is, the tibial insert 212 is restricted from rotating relative to the tibial tray 222 via corresponding locking mechanisms built into the tibial insert 212 and the tibial tray 222. The rotating platform tibial insert 214 is configured to couple with the tibial tray 224, which is a mobile or rotating tibial tray. That is, the tibial insert 212 is free to rotate about its stem relative to the tibial tray 224.

The femoral prosthesis 204 is embodied as a cruciate-retaining (CR) femoral prosthesis and, unlike the femoral prosthesis 202, does not include a posterior cam. The femoral prosthesis 204 is configured to articulate with either the tibial insert 216 or the tibial insert 218, each of which is devoid of a post and may be embodied as either a symmetrical or asymmetrical tibial insert, as described above. The tibial insert 216 is a fixed or non-rotating tibial insert and is configured to couple to the tibial tray 222. In contrast, the tibial insert 218 is a mobile or rotating insert and is configured to couple to the tibial tray 224.

In use, an orthopaedic surgeon may select the desired combination of orthopaedic knee prosthesis components and sizes based on the patient's boney anatomy (e.g., the extent of damage to or deterioration of the patient's knee joint) and/or the surgeon preferences. It should be appreciated that while only a single size of each of the above-described components of the orthopedic knee prosthesis system 200 is shown in FIG. 2A, the orthopedic knee prosthesis system 200 may include additional sizes of each of those components to accommodate the bony anatomies of various patients. The orthopedic knee prosthesis system 200 may also include versions of each of those components specifically designed for use in only the left or right knee joints.

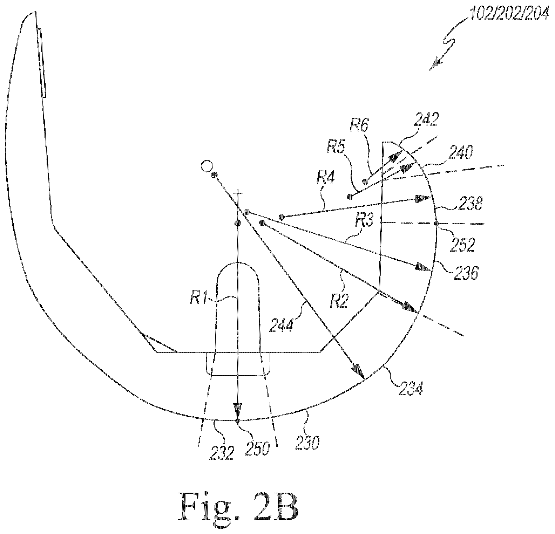

Referring now to FIG. 2B, the condyles of the illustrative femoral prostheses 102, 202, 204 each include a condyle surface 230, which is convexly curved in the sagittal plane. Illustratively, the condyle surface 230 is formed from a number of curved surface sections 232, 234, 236, 238, 240, and 242 each of which is tangent to the adjacent curved surface section. Each curved surface sections 232, 234, 236, 238, 240, and 242 contacts the tibial insert 106 (or one of the tibial inserts 212, 214, 216, 218) through different ranges of degrees of flexion. For example, the curved surface sections 232, 234 of the condyle surface 230 contact the tibial insert 106 during early flexion (including extension). The curved surface sections 236, 238 of the condyle surface 230 contact the tibial insert 106 during mid-flexion. And, the curved surface sections 240, 242 of the condyle surface 230 contact the tibial insert 106 during late flexion. Each curved surface sections 232, 236, 238, 240, and 242 is defined by a constant radius of curvature R1, R3, R4, R5, and R6, respectively. However, the curved surface section 234 is defined by a plurality of rays 244 (rather than a constant radius of curvature), such that the curved surface section 234 has a continuously decreasing radius of curvature and transitions gradually from the radius of curvature R1 of the curved surface section 232 to a radius of curvature R2, which is tangent to the curved surface section 236. Additional details regarding the sagittal curvature of the illustrative femoral prostheses 102, 202, 204 is set out in U.S. Patent Application Publication No. 2022/0008207. It is contemplated that other embodiments may use femoral prostheses having different sagittal curvatures, including sagittal curvatures defined by a single, constant radius.

When preparing for and performing a knee replacement surgery, an orthopaedic surgeon determines the type, size, and implant alignment of each prosthetic component to be implanted in the patient's knee joint. The surgeon may adjust or select the alignment of each prosthetic component along six degrees of freedom (medial-lateral, anterior-posterior, superior-inferior, flexion-extension, adduction-abduction, and internal-external) to achieve desired knee joint mechanics. When the type, size, and implant alignment are determined, the surgeon may resect the patient's femur and tibia (and, in some cases, patella) to prepare the bones to receive the prosthetic components. In the case of a total knee arthroplasty, the surgeon resects the patient's femur to create at least five planar surfaces and resects the patient's tibia to create a single planar surface. As described in more detail below, the surgeon may prepare the patient's bone using manual instruments, such as, for example, the ATTUNE^{®} INTUITION^{™} Instruments, which are commercially available from DePuy Synthes Products, Inc., and/or robotic devices, such as, for example, the VELYS^{™} Robotic-Assisted Solution, which is also commercially available from DePuy Synthes Products, Inc.

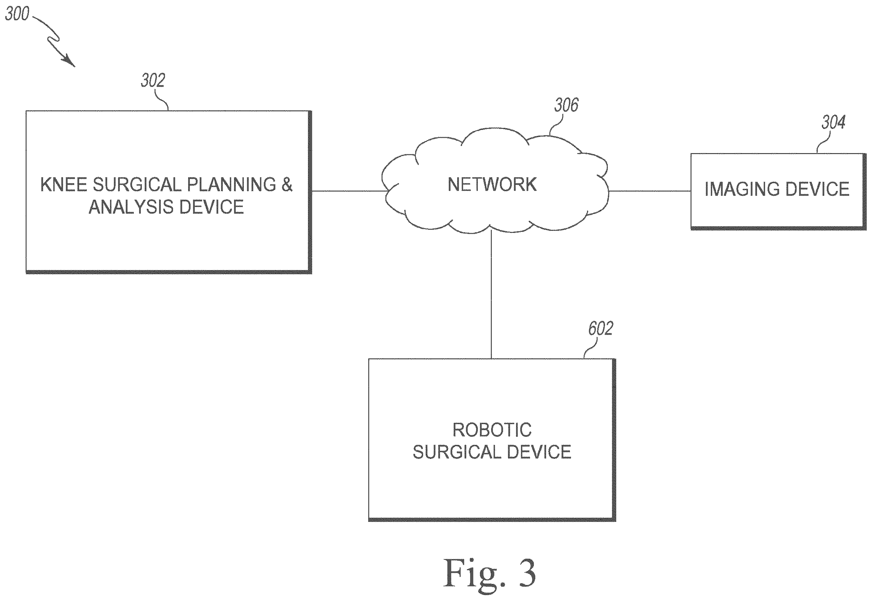

Referring now to FIG. 3, an illustrative computer system 300 for determining the positioning of a knee prosthesis, such as the knee prosthesis 100, includes a knee surgical planning and analysis device 302 and an imaging device 304 communicatively coupled to the analysis device 302 over a network 306. In use, an orthopedic surgeon may operate the analysis device 302 to select an implant size and type, determine a recommended implant alignment of the femoral and tibial prostheses 102, 104 of the knee prosthesis 100, and to prepare a surgical plan incorporating that recommended implant alignment to guide the preparation of the patient's bones to receive the knee prosthesis 100. As described in more detail below, the surgeon may execute the surgical plan using manual instruments and/or a robotic surgical device 602 programmed and configured in accordance with the surgical plan.

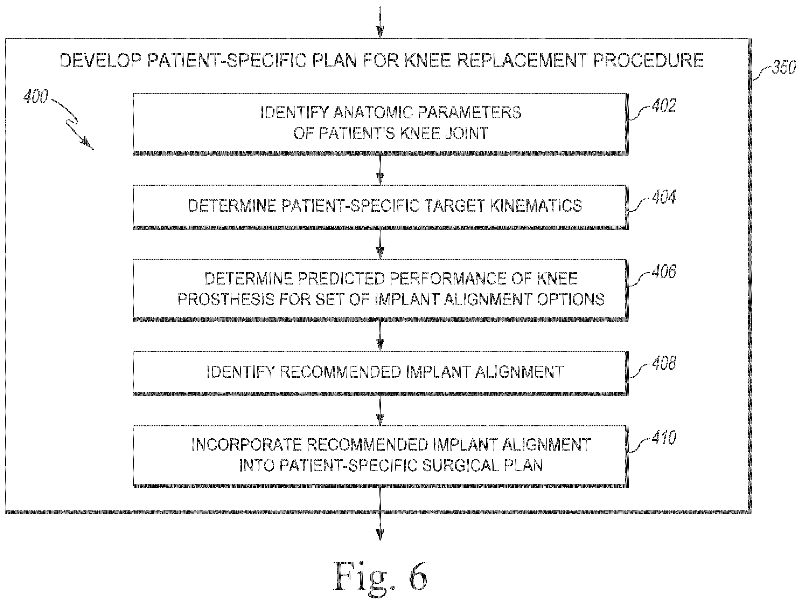

As described in more detail below in reference to FIG. 6, the analysis device 302 is configured to identify anatomical parameters that it can use to determine target kinematics specific to the patient. The anatomical parameters may be identified in an automated and/or surgeon-assisted fashion based on medical images, pre-operative models of the patient's knee joint, and/or landmarks located inter-operatively during the surgical procedure. The analysis device 302 also develops predictions of the performance of the knee prosthesis at various possible implant alignments based on the type and size of the knee prosthesis and the patient's anatomical parameters. Using mathematical models and rules-based decision criteria, the analysis device 302 is configured to identify the recommended implant alignment based on the patient-specific target kinematics and the predicted performance of the knee prosthesis and to incorporate the recommended implant alignment into a patient-specific surgical plan.

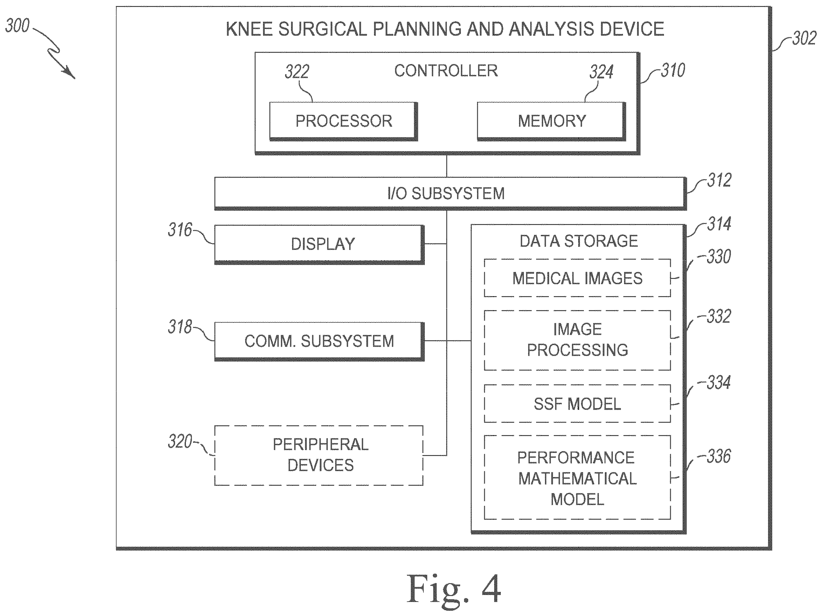

Referring now to FIG. 4, the analysis device 302 may be embodied as any type of computer device or system capable of performing the functions described herein. For example, the analysis device 302 may be embodied as a desktop computer, a surgical navigation computer, a laptop computer, a tablet computer, a smartphone, a mobile computer, a smart device, a wearable computer system, or other computer device or system. The knee surgical planning and analysis device 302 may be located separate from (e.g., in the orthopaedic surgeon's office) or within the surgical operating room.

As shown in FIG. 4, the illustrative analysis device 302 includes a controller 310, an input/output ("I/O") subsystem 312, a data storage 314, a display 316, a communication system 318 and, in some embodiments, one or more peripheral devices 320. Of course, the analysis device 302 may include additional or other components, such as those commonly found in a typical computer device, in other embodiments. Additionally, in some embodiments, one or more of the illustrative components may be incorporated in, or otherwise form a portion of, another component.

The controller 310 may be embodied as any type of controller, functional block, digital logic, or other component, device, circuitry, or collection thereof capable of performing the functions described herein. In illustrative embodiment, the controller 310 includes a processor 322 and a memory 324. The processor 322 may be embodied as any type of processor capable of performing the functions described herein. For example, the processor 322 may be embodied as a single or multi-core processor(s), digital signal processor, microcontroller, or other processor or processing/ controlling circuit. Similarly, the memory 324 may be embodied as any type of volatile and/or non-volatile memory or data storage capable of performing the functions described herein. In operation, the memory 324 may store various data and software used during operation of the analysis device 302 such as operating systems, applications, executable software, programs, libraries, drivers, and various data, which may be executed or otherwise used by the processor 322.

The controller 310 is communicatively coupled to other components of the analysis device 302 via the I/O subsystem 312, which may be embodied as circuitry and/or components to facilitate input/output operations between the controller 310 (e.g., the processor 322 and the memory 324) and the other components of the analysis device 302. For example, the I/O subsystem 312 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 312 may form a portion of a system-on-a-chip (SoC) and be incorporated, along with the controller 310 (e.g., the processor 322 and the memory 324) and other components of the controller 310, on a single integrated circuit chip. Additionally, in some embodiments, the memory 324, or portions of the memory 324, may be incorporated into the processor 322.

The data storage 314 may be embodied as any type of device or devices configured for short-term and/or long-term storage of data such as, for example, solid-state drives, hard disk drives, memory devices and circuits, memory cards, non-volatile flash memory, or other data storage devices. In the illustrative embodiment, the data storage 314 stores various data used by the analysis device 302 to perform the functions described herein. For example, the data storage 314 may store one or more medical images 330 of the patient. The medical images 330 may be generated by the imaging device 304 and transmitted to the analysis device 302 over the network 306 for local storage in the data storage 314. As described in more detail below, the medical images may be embodied as X-ray images, computed tomography (CT) images, magnetic resonance imaging (MRI) images, three-dimensional models, and/or other medical images, models, or related data of the patient's knee joint.

The data storage 314 may also store one or more image processing modules 332, which may be embodied as one or more mathematical models or algorithms (e.g., a machine learning algorithm) capable of analyzing the medical images and determining associated anatomical parameters, including anatomical landmarks. As described in more detail below, the analysis device 302 may determine the anatomical parameters in an automated fashion using the landmark models 332 and/or determine the anatomical parameters in a manual fashion based on annotations of the medical images received from the orthopaedic surgeon.

Additionally, the data storage 314 may store at least one statistical shape-function model 334. As described in greater below, the statistical shape-function model 334 is a mathematical model that matches or correlates the shape of the patient's knee bones (femur, tibia, patella) to a library of "healthy" knee bones and their associated kinematics. The statistical shape-function model 334 is configured to determine target kinematics for the patient's knee joint based on the kinematics associated with similarly-shaped "healthy" knee bones.

The data storage 314 may further store a performance mathematical model 336. As described in more detail below, the performance mathematical model 336 is a mathematical model of joint kinematics and mechanics produced by each orthopaedic knee prosthesis (i.e., the type and size of the orthopaedic knee prosthesis) across a number of implant alignments (i.e., combinations of positions and orientations of the tibial tray 104 and the femoral prosthesis 102 when implanted) using anatomical parameters as an input. In this way, the mathematical model 336 may quickly generate or produce the joint kinematics and mechanics for a particular orthopaedic knee prosthesis at a particular alignment because such calculations have been previously computed. In the illustrative embodiment, the performance mathematical model 336 is embodied as a linear response model, but it may be embodied as other types of models in other embodiments. By way of example, in some embodiments, the performance mathematical model 336 is embodied as a machine learning model.

The display 316 may be embodied as any type of display capable of displaying information to a user (e.g., the orthopaedic surgeon) of analysis device 302. For example, the display 316 may be embodied as a liquid crystal display (LCD), a light emitting diode (LED) display, an organic light emitting diode (OLED), a cathode ray tube (CRT) display, a plasma display, and/or other display device. In some embodiments, the display 316 may include a touchscreen, which may be configured to receive input from the orthopaedic surgeon based on a tactile interaction. Additionally, in some embodiments, the display 316 or a duplicate display 316 may be separate from the analysis device 302 but communicatively coupled thereto.

The communication subsystem 318 may be embodied as any type of communication circuit, device, or collection thereof, capable of enabling communications between the analysis device 302 and the imaging device 304 and/or other devices of the computer system 300. To do so, the communication subsystem 318 may be configured to use any one or more communication technologies (e.g., wireless or wired communications) and associated protocols (e.g., Ethernet, Bluetooth^{®}, Wi-Fi^{®}, WiMAX, LTE, 5G, etc.) to effect such communication.

The one or more peripheral device(s) 320 may include any number of additional peripheral or interface devices, such as other input/output devices, storage devices, and so forth. The particular devices included in the peripheral device(s) 320 may depend on, for example, the type and/or intended use of the analysis device 302.

Returning to FIG. 3, the imaging device 304 may be embodied as any type of device or collection of devices capable of pre-operatively and/or intra-operatively generating medical images of the boney anatomy of the patient. In the illustrative embodiments, the imaging device 304 is embodied as a CT imaging machine capable of generating CT medical images. However, in other embodies, the imaging device 304 may be embodied an imaging device capable of generating X-Ray medical images and/or three-dimensional models. It is contemplated that the imaging device 304 need not be used in all embodiments.

The network 306 may be embodied as any type of communication network capable of facilitating communication between the analysis device 302 and the imaging device 304 (and other components of the computer system 300). As such, the network 306 may include one or more networks, routers, switches, gateways, computers, and/or other intervening devices. For example, the network 306 may be embodied as or otherwise include one or more local or wide area networks, cellular networks, publicly available global networks (e.g., the Internet), an ad hoc network, a short-range communication network or link, or any combination thereof. It should also be appreciated that various modules of the analysis device 302, such as, for example, the models 334, 336 may be network- or cloud-based and accessed remotely through a properly configured local computing system. It should also be appreciated that the modules of the analysis device 302 may also be accessed by or incorporated into the robotic surgical device 602.

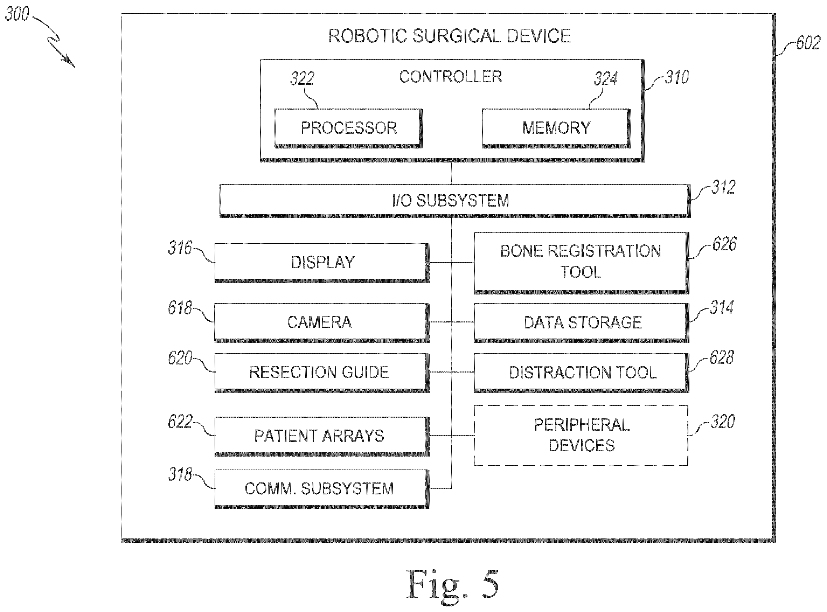

Referring now to FIG. 5, the illustrative robotic surgical device 602 includes a number of components that are similar to the analysis device 302. The same reference numbers will be used to refer to those components, and the description of those components will not be duplicated in reference to the robotic surgical device 602. The robotic surgical device 602 includes a controller 310, an input/output ("I/O") subsystem 312, a data storage 314, a display 316, a communication system 318, and, in some embodiments, one or more peripheral devices 320. The robotic surgical device 602 also includes a camera 618, a resection guide 620, one or more patient arrays 622, a bone registration tool 626, and a bone distraction device 628. Of course, the surgical device 602 may include additional or different components, such as those commonly found in a typical computer device. Additionally, in some embodiments, one or more of the illustrative components may be incorporated in, or otherwise form a portion of, another component. It should also be appreciated that, in some embodiments, some of these components may be omitted. For example, some embodiments of the robotic surgical device 602 may not include a bone distraction device 628 or a resection guide 620. In such embodiments, steps utilizing the omitted components may be omitted or modified to utilize other components.

The camera 618 may be embodied as any type of camera or imaging device capable of capturing images of the patient's boney anatomy and, in particular, visual markers attached to the patient's boney anatomy. For example, in some embodiments, the camera 618 may be embodied as an infrared camera. Additionally, in some embodiments, the camera 618 may include an array of cameras to assist in identifying the location of the patient's boney anatomy in three-dimensional space.

The resection guide 620 may be embodied as any type of guide controllable by the surgical device 602 to control the resection of the patient's boney anatomy. For example, the resection guide 620 may be embodied as a robotic arm attached to, or attachable to, an orthopaedic saw to position and control the cutting plane of the saw. In other embodiments, the resection guide 620 may include an automated cutting tool that is operated by the controller 310. In still other embodiments, the resection guide 620 may be embodied as, or otherwise include, one more cutting blocks or guides. In yet other embodiments, the resection guide 620 may be implemented virtually using augmented reality or another method for indicting planned cutting planes to the surgeon.

The patient arrays 622 are embodied as various brackets and/or other devices configured to attach to the patient's boney anatomy. Once secured, the patient arrays 622 are detectable by the camera 618 to allow the surgical device 602 to monitor the movement of the patient's leg in three-dimensional space such that the analysis device 302 can determine kinematics of the patient's knee joint based on such movements, as described in more detail below.

The bone registration tool 626 is embodied as a stylus or pointer that may be used by the surgeon to contact various locations on the patient's tibia and femur to acquire anatomical parameters (e.g., landmarks and bone axes). The registration tool 626 is detectable by the camera 618 to record the position and orientation of desired anatomical parameters.

The robotic surgical device 602 also includes a bone distraction tool 628 configured to adjust the distance between the distal end of the femur and the proximal end of the tibia. The distraction tool 628 may be a manual instrument such as, for example, the instrument shown and described in U.S. Patent No. 10,945,777. In other embodiments, the distraction tool 628 may be an electromechanical tool controlled by the analysis device 302 to adjust the distance between the patient's bones.

Referring now to FIG. 6, the analysis device 302 includes a module 350 that is configured to execute a method 400 for developing a patient-specific surgical plan for the patient. For example, the method 400, or portions thereof, may be embodied as a set of executable instructions stored on the analysis device 302 and executable by the analysis device 302. It should also be appreciated that the operations of the module 350 and the method 400 may be performed by one or more components of the analysis device 302 and/or devices communicatively coupled to the analysis device 302, including, for example, the surgical device 602. In some embodiments of the method 400, developing a patient-specific surgical plan for a patient may be embodied as modifying portions of an existing patient-specific surgical plan previously developed for the patient.

In block 402 of the method 400, the analysis device 302 determines various anatomical characteristics or parameters of the patient's knee joint. The determined or identified anatomical parameters may be embodied as any anatomical landmark or other parameter (e.g., relative dimensions of the patient's boney anatomy) that facilitates or improves the determination of the target kinematics and/or the predicted performance of the knee prosthesis. The particular landmarks used may depend on various factors such as the patient's bony anatomy, the size and type of knee prosthesis 100, and/or other factors. For example, in the illustrative embodiment, the anatomical parameters used include the center and radius of the femoral medial condyle, the center and radius of the femoral lateral condyle, the posterior slope(s) of the tibial plateaus (medial and lateral), the varus-valgus angle of the tibia articular surface, the rotational angle to the tibial tuberosity, and the rotational angle to the fibular head. In other embodiments, the anatomical parameters may additionally or alternatively include any anatomical landmarks of the tibia center, the femur center, the femoral epicondyles (medial and lateral), and the malleoli, including the posterior cruciate ligament (PCL) attachment site and the most distal and posterior point(s) on the femoral medial and/or lateral condyle(s), by way of example. The anatomical parameters may also include axes such as the tibial sagittal axis, Whiteside's line, and the femoral and tibial mechanical axes. Additional anatomical parameters may facilitate the determination or calculation of various coordinate systems (e.g., an anatomic femoral coordinate system, an anatomic tibial coordinate system, and/or a femoral condylar coordinate system).

The anatomical parameters may be derived from intra-operative bone registration (as described in greater detail below), medical images of the patient's pre-operative anatomy, or a combination of bone registration and medical images. The anatomical parameters are determined with the patient's knee joint positioned in various functional positions. The analysis device 302 may receive any type and number of suitable medical images that facilitate the determination of the patient's target kinematics, as described in more detail below.

The analysis device 302 also determines, in block 404, patient-specific target kinematics. For example, in the illustrative embodiment, the analysis device 302 determines the patient-specific target kinematics based on a patient-generic statistical shape-function model using the identified anatomical parameters (or a sub-set thereof) as inputs. In other embodiments, the patient-specific target kinematics may be measured directly from the patient (e.g., intra-operatively) by moving the patient's leg through a range of motion while the camera 618 observes the patient arrays 622 attached to the patient's femur 110 and tibia 112, allowing the surgical device 602 to monitor the movements of the patient's leg in three-dimensional space such that the analysis device 302 can determine kinematics of the patient's knee joint based on such movements. As a result of block 404, the analysis device 302 now possesses knowledge of the position of the femur 110 and the tibia 112 relative to one another at a variety of flexion angles throughout the range of motion (which positions can also be translated to the global reference frame of the camera 618, as desired).

The statistical shape-function model illustratively used in block 404 is embodied as a mathematical model that uses the anatomical parameters to match or correlate the shape of the patient's knee bones, as defined by the anatomical parameters, to a library of "healthy" knee bones. The library of knee bones may be established based on a pool of individuals having healthy knee joints. Illustratively, target kinematics determined by the statistical shape-function model include the varus-valgus angle, the internal-external rotation, the medial-lateral translation, the anterior-posterior translation, and the superior-inferior translation of the patient's bones through a range of flexion. In other embodiments, the target kinematics may include additional or other kinematic parameters of the patient's knee joint. By matching or correlating anatomic characteristics or parameters of the patient's knee bones to a library of "healthy" joints and associated kinematics, the "healthy" or non-damaged/non-deteriorated kinematics of the patient's knee joint is determined, providing patient-specific target kinematics to be achieved via the knee prosthesis implantation.

The target kinematics include properties related to the location/position, orientation, or motion of patient's bones without reference to the forces or loading on the patient's bones. In the illustrative embodiment, the target kinematics includes a patient-specific target ligament elongation across a range of flexion for one or more ligaments of the patient's knee joint. For example, in the illustrative embodiment, the analysis device 302 may determine a target ligament elongation for the medial collateral ligament (MCL), the lateral collateral ligament (LCL), the posterior cruciate ligament (PCL), the anterolateral ligament, and the posterior capsule of the patient's relevant knee joint. The femoral and tibial attachment sites of each of these ligaments may be identified in block 402 (e.g., using imaging or intra-operative measurements) or may be determined from other identified anatomical parameters in block 404 (e.g., using a statistical shape model). The analysis device 302 then applies the target kinematics, using transformation matrices, to determine the relative position of patient's femur and tibia in a number of positions throughout a range of flexion and calculates a distance between the femoral and tibial attachment sites for each ligament in each of the number of positions. These calculations provide an indication of the target length of each ligament across the range of flexion.

As shown in FIG. 6, the analysis device 302 then proceeds to determine the predicted performance of a knee prosthesis across a number of implant alignment options in block 406. The term "implant alignment" as used herein refers to the position and orientation of the femoral prosthesis on the distal end of the patient's femur and the position and orientation of the tibial tray on the proximal end of the patient's tibia. In some embodiments, implant alignment may also refer to the position and orientation of a patella prosthesis. As noted above, the position and orientation of each prosthetic component can be varied in up to six degrees of freedom (medial-lateral, anterior-posterior, superior-inferior, flexion-extension, adduction-abduction, and internal-external). The analysis device 302 predicts the performance of the knee prosthesis over a range of flexion when the knee prosthesis is implanted at each implant alignment in a set of implant alignment options. It will be appreciated that the number of implant alignments in a set of implant alignment options will depend on the permitted values for each of the degrees of freedom for each of the prosthetic components of the knee prosthesis. For example, constraining (e.g., decreasing the range of) the permitted values for the one or more degrees of freedom of one or more prosthetic components of the knee prosthesis (e.g., limiting or even fixing the superior-inferior position of the femoral prosthesis) will decrease the number of implant alignments in the set of implant alignment options. As described in more detail below, the permitted values of the degrees of freedom may be constrained in a number of different ways, including, but not limited to, predetermined system constraints, surgeon preferences, and/or initial evaluation algorithms that determine narrowed sets of implant alignment options meeting one or more specified criteria.

As described in greater detail below, the predicted performance in the illustrative embodiment includes the predicted kinematics of the knee prosthesis when implanted in the knee joint. The predicted performance may also include the predicted mechanics of the knee prosthesis when it is implanted. The predicted mechanics may include the predicted forces or loading on the knee prosthesis or patient's bones for each implant alignment. The predicted mechanics may also include the interfacial stresses and contact stresses that may be derived from the predicted forces. Such predictions may also include indications of implant alignments presenting greater or lesser risk of difficulties with the knee prosthesis, including, for example, bonding delamination between the components of the prosthesis and the patient's bones and wear performance of the tibial insert.

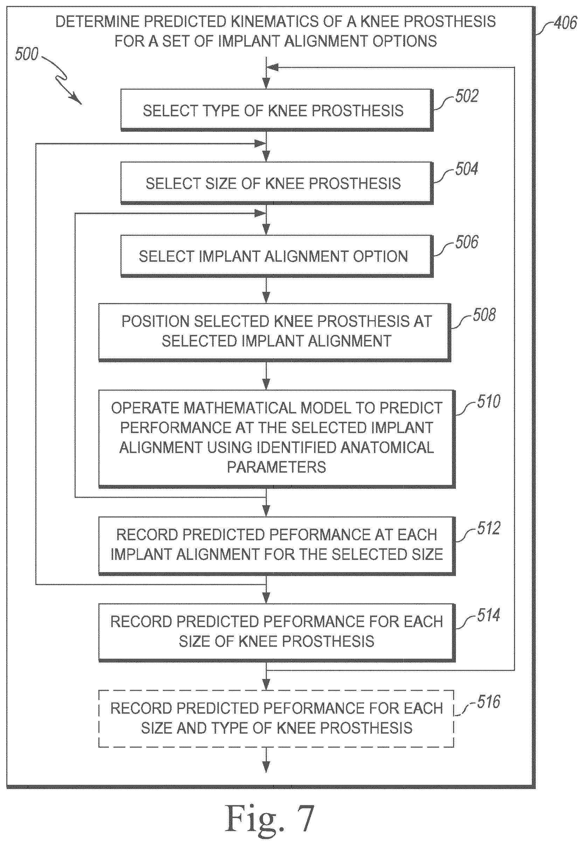

Referring now to FIG. 7, the analysis device 302 may predict the performance of the knee prosthesis in block 406 via a process 500. In process 500, the anatomical parameters identified in block 402 are provided for use in determining the predicted performance. The analysis device 302 also selects a type of knee prosthesis (for at least the tibial tray, tibial insert, and femoral prosthesis) in process step 502 and a size of knee prosthesis (for at least the tibial tray, tibial insert, and femoral prosthesis) in step 504. It should be appreciated that the orthopaedic surgeon may select the type and size from a menu of available types and sizes or otherwise provide those selections to the analysis device 302. The analysis device 302 may also be configured to determine predictions for each possible combination of type and size of each component of the knee prosthesis available in the implant system 200.

In process step 506, the analysis device 302 selects an implant alignment option from among the set of implant alignment options under consideration. In the illustrative embodiment, the implant alignment of the knee prosthesis 100 is defined by nine degrees of freedom for the positions and orientations of the femoral and tibial prostheses of the knee prosthesis. These nine degrees of freedom are femoral flexion-extension angle, femoral varus-valgus rotation, femoral internal-external rotation, femoral superior-inferior resection distance, femoral anterior-posterior position, tibial slope, tibial varus-valgus rotation, tibial internal-external rotation, and tibial insert thickness. However, in other embodiments, additional, fewer, and/or different degrees of freedom may be used for the implant alignment options.

After selecting one implant alignment from the set of implant alignment options, the analysis device 302 continues to block 508 and "positions" the selected prosthetic components at the selected implant alignment in a performance mathematical model 336. To do so, the analysis device 302 accesses configuration data for each prosthetic component, including the size, shape, and geometry of the selected femoral prosthesis and the selected tibial tray and insert components. The analysis device 302 inputs that data into the performance mathematical model 336 with the identified anatomical parameters to configure the mathematical model to make patient-specific predictions on the performance of the knee prosthesis when positioned at the selected implant alignment in the patient's knee joint. In that way, the analysis device 302 virtually implants the selected prosthetic components into the patient's knee joint at the selected implant alignment.

Next, in block 510, the analysis device 302 operates the performance mathematical model 336 to predict the performance of the selected prosthetic components. The performance mathematical model 336 in the illustrative embodiment is a linear response model, which is derived from pre-calculating the mathematical model for each combination of type and size of orthopaedic knee prosthesis, combination of alignment parameters, and combination of anatomical parameters. In that way, the linear response model defines pre-calculated performance for each combination of input parameters, which greatly increases the computation speed of the linear response model relative to performing such calculations during "run time." As such, the model is configured to predict performance of the selected orthopaedic knee prosthesis 100.

As described above, the linear response model 336 includes a model of joint kinematics (and, in some embodiments, mechanics) such that the model 336 may determine, for a range of flexion, the predicted kinematics (and, in some embodiments, mechanics) of the selected prosthetic components at the selected implant alignment based on the size and type of prosthetic components and the identified anatomical parameters. In the illustrative embodiment, the predicted kinematics determined in block 510 includes a predicted ligament elongation across the range of flexion for the one or more ligaments of the patient's knee joint. In doing so, as described above, the analysis device 302 may predict the ligament elongation for the medial collateral ligament (MCL), the lateral collateral ligament (LCL), the posterior cruciate ligament (PCL), the anterolateral ligament, and the posterior capsule of the patient's relevant knee joint.

After the analysis device 302 has determined the predicted performance in block 510 for the selected prosthetic components at the selected implant alignment, the process 500 may repeat steps 506, 508, 510 to predict performance of the selected prosthetic components at each of the other implant alignments in the set of implant alignment options under consideration. To do so, the analysis device 302 may change the implant alignment along one or more degrees of freedom (e.g., tibial anterior-posterior slope to re-position the tibial prosthesis on the proximal end of the patient's tibia) before repeating step 510 to obtain another prediction of performance for the new implant alignment. The analysis device 302 continues this process, repeating steps 506, 508, 510 until a performance prediction has been generated for each implant alignment of the set of implant alignment options. The performance prediction for each implant alignment (e.g., the predicted kinematics and/or the predicted ligament elongations) is recorded or stored in process step 512.

The analysis device 302 may also repeat steps 504, 506, 508, 510 to predict performance for different sizes of prosthetic components. The performance prediction at each implant alignment for each size of prosthetic component, including the predicted kinematics and the predicted ligament elongations, is recorded or stored in process step 514. If desired, the analysis device 302 may also repeat steps 502, 504, 506, 508, 512 to predict performance of all of the possible combinations of different types and sizes of prosthetic components of implant system 200. The performance prediction at each possible implant alignment for each possible knee prosthesis (including predicted kinematics and/or predicted ligament elongations) may be recorded or stored in process step 516.

After determining the predicted performance of the knee prosthesis for each desired type, size, and implant alignment option in block 406, the method 400 advances to block 408 (see FIG. 6) in which the analysis device 302 identifies one or more recommended implant alignments for the selected orthopaedic knee prosthesis 100. To do so, the analysis device 302 is configured to evaluate and compare the target kinematics determined in block 404 with the predicted performance of the knee prosthesis. In the illustrative embodiment, the analysis device 302 may calculate a cost function for each implant alignment of the orthopaedic knee prosthesis 100 across the range of flexion (illustratively, 0 degrees to 120 degrees of flexion) and select the recommended implant alignment of the orthopaedic knee prosthesis 100 based on that results of that cost function. For example, the analysis device 302 may determine summed absolute differences over the range of flexion between the target and implanted lengths of the medial collateral ligament (MCL), the lateral collateral ligament (LCL), and the posterior cruciate ligament (PCL) of the patient's knee joint according to the cost function: $Cost Function = {\sum }_{k = 0 °}^{120 °} \left(\begin{matrix}\left|MCL {Length}_{Target}-MCL {Length}_{Implanted}\right| + \\ \left|LCL {Length}_{Target}-LCL {Length}_{Implanted}\right| + \\ \left|PCL {Length}_{Target}-PCL {Length}_{Implanted}\right|\end{matrix}\right)$

In the illustrative embodiment, the analysis device 302 selects the implant alignment of the orthopaedic knee prosthesis 100 having the lowest computed cost function (i.e., the implant alignment in which the length of the relevant ligaments are most closely aligned with the length of the corresponding target ligaments) as the recommended implant alignment. In other embodiments, the analysis device 302 may select all implant alignments having a computed cost function below a threshold as the recommended implant alignment(s). In still other embodiments the analysis device 302 may select or determine the recommended implant alignment(s) based on a set of decision rules. The decision rules may define particular criteria on which the recommendation should be based (e.g., a requirement for a particular ligament length at a particular degree of flexion). The decision rules may require the analysis device 302 to evaluate and weigh the implant alignment options based on other aspects of the predicted kinematics such as, for example, the degree of internal-external rotation and aspects of mechanics such as, for example, the predicted forces and stresses on the knee prosthesis. In some embodiments, block 408 may also account for preferences specified by the surgeon, such as constraints on one or more degrees of freedom for the implant alignment, a preference between implant sizes when two adjacent sizes have the same or similar performance metrics, or the like.

After the analysis device 302 has selected the recommended implant alignment(s) of the orthopaedic knee prosthesis 100, the method 400 advances to block 410 to incorporate one or more of the recommended implant alignment(s) into a patient-specific surgical plan 800. In some embodiments, block 410 may involve preparing a new surgical plan 800 that includes the recommended implant alignment(s). In other embodiments, block 410 may involve modifying portions of an existing surgical plan 800 that was previously developed for the patient. The surgical plan 800 may be displayed on the display 316 for review by the surgeon on the analysis device 302 prior to surgery or displayed intraoperatively on the surgical device 602. The surgical plan 800 may also be embodied in hardcopy form.

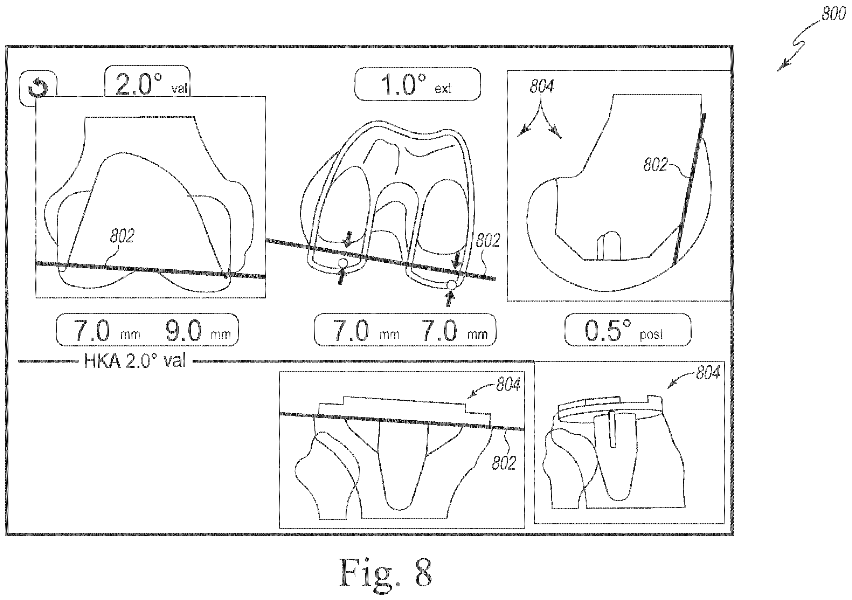

As shown in FIG. 8, the patient-specific surgical plan 800 may include a graphical representation of the resection planes 802 on the patient's bones, which shows the angle of each plane and amount of bone to be removed from each portion of the patient's bones to place the prosthetic components at a selected implant alignment (e.g., the recommended implant alignment). The surgical plan 800 may also include visualizations 804 of the prosthetic components positioned on the patient's bones at the selected implant alignment. The surgical plan may also include indicia of the recommended size and type of prosthetic components, as well as other information to guide the surgical procedure.

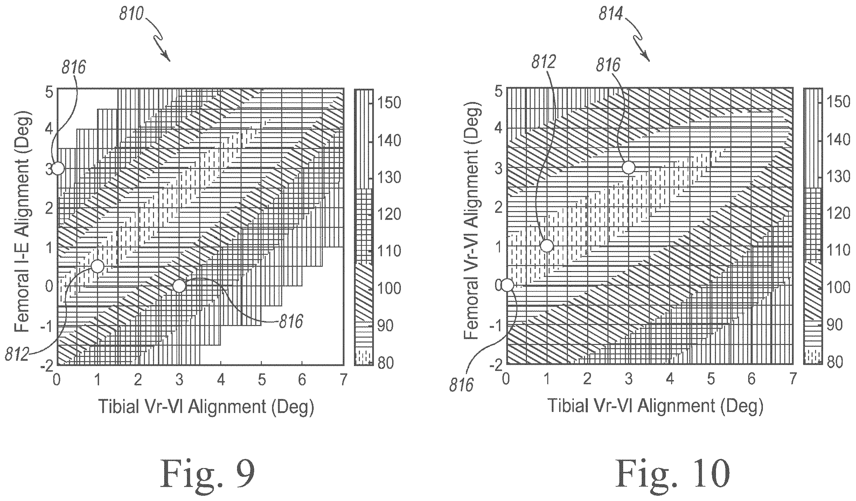

In some embodiments, the surgical plan 800 may include graphical representations about the expected performance of the selected knee prosthesis at a number of alignment options, including one or more recommended implant alignment(s). For example, as shown in FIG. 9, the surgical plan 800 may include a heat map 810 illustrating the output of the ligament length cost function discussed above for various combinations of femoral internal-external rotations and tibial varus-valgus slopes, with an indication 812 of a recommended implant alignment. As another example, FIG. 10 shows a heat map 814 illustrating the output of the ligament length cost function for various combinations of femoral varus-valgus rotations and tibial varus-valgus slopes, with an indication 812 of a recommended implant alignment, which may additionally or alternatively be included in the surgical plan 800. The heat maps 810, 814 may include a scale for the output of the ligament length cost function, which is illustrated on the right hand side of each of FIGS. 9-10. The heat maps 810, 814 may also include indications 816 of other typical alignment techniques, such as, for example, mechanical alignment or anatomic alignment, to provide the surgeon with a visual comparison of the patient-specific alignment with those other alignment techniques. It should be appreciated that the analysis device 302 may additionally or alternatively display other heat maps illustrating the output of the ligament length cost function for other input variables, such as the patient's planned flexion and/or extension gaps, as well as different alignment combinations. In this way, the surgical plan 800 may provide visual guidance to the orthopaedic surgeon of the kinematics of the orthopaedic knee prosthesis at various alignments.

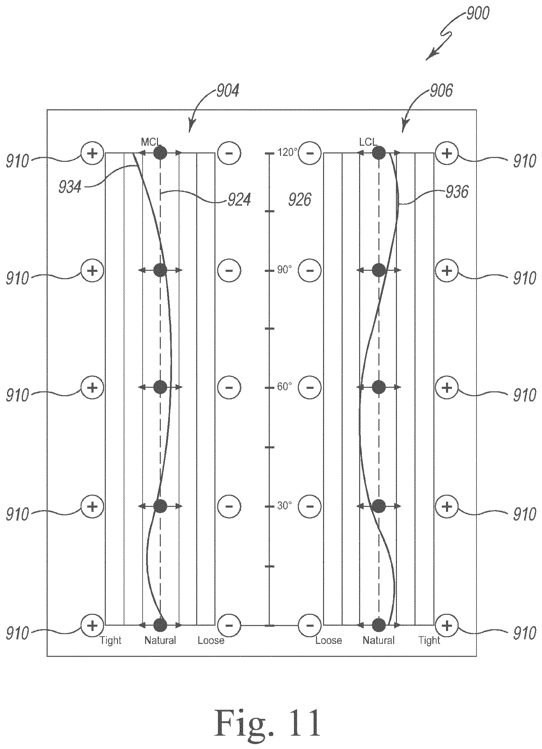

The orthopaedic surgeon may adjust the implant alignment using the devices 302, 602 as needed, including during pre-operative activities or intra-operatively. For example, the devices may be configured to display a user interface including the surgical plan 800. The user interface may be configured to receive adjustments from the surgeon for any of the alignment parameters, including, for example, the size and type of prosthetic components, the tibial slope, the varus-valgus slope of the tibia or femur, the cutting depths, internal-external rotation, and tibial insert thickness. The device may also be configured to display a user interface 900 as shown in FIG. 11. The interface 900 includes an MCL elongation portion 904 and an LCL elongation portion 906. For example, the illustrative MCL elongation portion 904 and LCL elongation portion 906 include controls 910 that the orthopaedic surgeon may manipulate to adjust the laxity of the corresponding ligament at defined degrees of flexion. The dashed lines 924, 926 of the MCL elongation portion 904 and the LCL elongation portion 906 indicate the target ligament elongations, while the solid lines 934, 936 indicate the predicted ligament elongations. As the orthopaedic surgeon adjusts the alignment parameters and/or the ligament elongations 904, 906, the analysis device 302 performs steps 402, 404, 406, 410 to update the predicted performance of the knee prosthesis and patient-specific plan based on the adjustments.

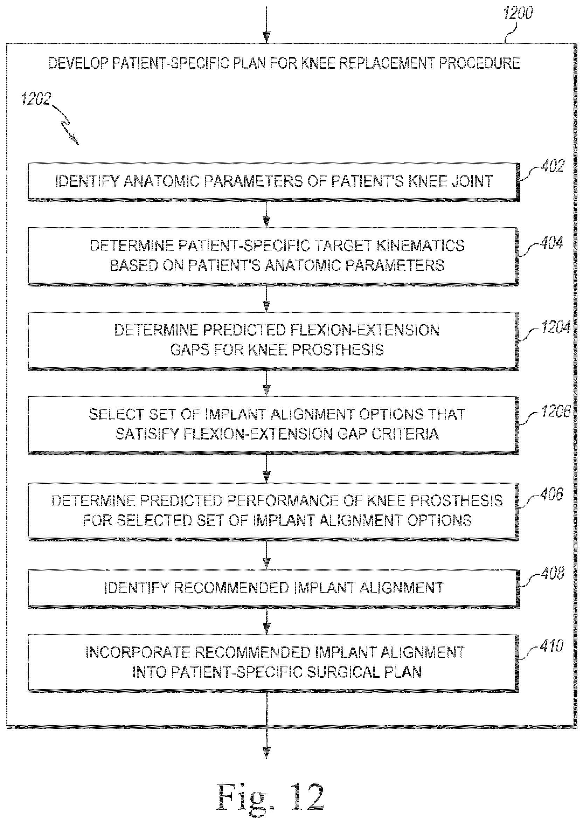

Referring now to FIG. 12, an alternative module 1200 that may be included in the analysis device 302 (in place or in addition to the module 350) is illustrated. The module 1200 is configured to execute a method 1202 for developing a patient-specific surgical plan 800 for the patient. For example, the method 1202, or portions thereof, may be embodied as a set of executable instructions stored on the analysis device 302 and executable by the analysis device 302. It should also be appreciated that the operations of the module 1200 and the method 1202 may be performed by one or more components of the analysis device 302 and/or devices communicatively coupled to the analysis device 302, including, for example, the surgical device 602. In some embodiments of the method 1202, developing a patient-specific surgical plan for a patient may be embodied as modifying portions of an existing patient-specific surgical plan previously developed for the patient.

In block 402 of the method 1202, the analysis device 302 identifies various anatomical characteristics or parameters of the patient's knee joint. Block 402 of method 1202 may include any or all of the features described above for block 402 of method 400 (FIG. 6). For example, the anatomical parameters may be derived from intra-operative bone registration (as described in greater detail below), medical images of the patient's pre-operative anatomy, or a combination of bone registration and medical images.

After the anatomical parameters have been identified, the method 1202 proceeds to block 404 in which patient-specific target kinematics are determined. Block 404 of method 1202 may include any or all of the features described above for block 404 of method 400 (FIG. 6). For example, the analysis device 302 may determine the patient-specific target kinematics based on a patient-generic statistical shape-function model using the identified anatomical parameters (or a sub-set thereof) as inputs. In some embodiments, the target kinematics include a target length for each of one or more ligament of the patient's knee joint, as described above. In other embodiments, patient-specific target kinematics may be measured directly from the patient (e.g., intra-operatively) by moving the patient's leg through a range of motion while monitored by the surgical device 602 such that the analysis device 302 can determine kinematics of the patient's knee joint based on such movements.

When implementing module 1200, the analysis device 302 is also configured to perform block 1204 of method 1202. During block 1204, the analysis device 302 determines one or more predicted gaps 116 for a selected knee prosthesis 100 when implanted in the knee joint at each of an initial set of implant alignment options. As discussed above, the position and orientation of each prosthetic component (i.e., the implant alignment) can be varied in up to six degrees of freedom. A set of implant alignment options includes the implant alignments reflecting each combination of all the possible values of each degree of freedom for the prosthetic component(s), within whatever constraints are applicable to that set. As such, the initial set of implant alignment options used in block 1204 reflects an initial set of degrees of freedom for positioning of the femoral prosthesis 102 relative to the femur 110 and for positioning of the tibial tray 104 relative to the tibia 112.

In one illustrative embodiment of block 1204, the analysis device 302 virtually "implants" or positions the selected prosthetic components at each implant alignment in the initial set of implant alignment options and calculates a predicted gap 116 corresponding to that implant alignment. For instance, for a particular implant alignment option, the femoral prosthesis 102 is virtually fixed relative to the femur 110, while the tibial prostheses 104, 106 are virtually fixed relative to the tibia 112. The analysis device 302 can then determine a position of the femoral prosthesis 102 relative to the tibial prostheses 104, 106 (or to a part of the tibia 112 itself, such as the planned tibial resection plane) at different points in the range of motion of knee joint by applying one or more transformation matrices to the target kinematics of the knee joint. For each flexion angle of interest (e.g., 0 degrees, 90 degrees), the analysis device 302 can then measure a smallest distance between the medial condyle of the femoral prosthesis 102 and the tibia 112 (or one of the tibial prostheses 104, 106) to determine a predicted medial-side gap 116a and a smallest distance between the lateral condyle of the femoral prosthesis 102 and the tibia 112 (or one of the tibial prostheses 104, 106) to determine a predicted lateral-side gap 116b. The analysis device 302 can repeat this process to determine at least one predicted gap 116 for each implant alignment in the initial set of implant alignment options.

As discussed above, the gap(s) 116 may be defined in numerous ways and any of those gap(s) 116 can be predicted in block 1204. For instance, in some embodiments, rather than searching for the point on a virtually-implanted femoral prosthesis 102 that is closest to the tibia 112 at each flexion angle of interest for each implant alignment option, the analysis device 302 can instead utilize a predetermined point on the sagittal curvature of the femoral prosthesis 102 corresponding to each flexion angle of interest. By way of example, with reference to FIG. 2B, the point 250 could be used for determining a predicted gap 116 at 0 degrees flexion, while the point 252 could be used for determining a predicted gap 116 at 90 degrees flexion. When the femoral prosthesis 102 is virtually implant by the analysis device, the location of each such predetermined point relative to the patient's bones is readily identifiable based on the known geometry of the femoral prosthesis 102, allowing for more efficient determination of predicted gap(s) 116. In some embodiments, this information can be used to train a mathematical model (e.g., a linear response model) to output predicted gap(s) 116 for the initial set of implant alignment options, using prosthesis size and patient-specific anatomical parameters and/or kinematics (from blocks 402 and/or 404) as inputs. It is also contemplated that block 1204 may involve predicting gap(s) 116 for any number of positions of the knee joint throughout the range of motion. By way of example, in some embodiments, the analysis device 302 may predict gap(s) 116 for each implant alignment when the knee joint is in extension, in mid-flexion, and in late-flexion.

After block 1204, the method 1202 proceeds to block 1206 in which the analysis device 302 selects a set of implant alignment options from among the initial set of implant alignment options analyzed in block 1204 (e.g., the selected set may be a subset of the initial set). In the illustrative embodiment, block 1206 involves determining which implant alignments from the initial set of implant alignment options resulted in predicted gaps 116 meeting one or more flexion-extension gap criteria. It is contemplated that additional and/or different criteria may be used for narrowing a larger, initial set of implant alignment options down to a smaller, selected set of implant alignment options. For instance, in other embodiments, the analysis device may use other performance predictions and/or surgeon preferences to narrow the set of implant alignment options in block 1206. As one example, a surgeon may constrain one or more degrees of freedom (e.g., through preference settings) such that only implant alignments in which the tibial tray is perpendicular to the mechanical axis are included in the set of implant alignment options selected in block 1206.

In the illustrative embodiment of method 1202, the analysis device 302 performs block 1206 by calculating a performance metric for each implant alignment option of the initial set of implant alignment options based on the predicted gap(s) 116 determined in block 1204. By way of example, block 1206 may involve comparing the predicted gap(s) 116 determined for each implant alignment option to one or more reference values. The reference value(s) may be constant or may vary with flexion. The reference value(s) may be user-defined, such that they reflect desired gap(s) 116 for implanted knee prosthesis 100. The analysis device 302 may implement block 1206 by calculating a difference between the predicted gap(s) 116 and the reference value(s) at each flexion angle of interest and then summing the absolute values of those differences to arrive at the performance metric for each implant alignment option. In some embodiments, the analysis device may calculate separate performance metrics for the medial side and the lateral side at each implant alignment option. In some embodiments, the medial and lateral performance metrics can be summed to create a single performance metric for the implant alignment option. The foregoing performance metric calculations may be implemented as a cost function to find an optimal implant alignment (or alignments) across a set or subset of implant alignment options. For instance, for each subset of implant alignment options sharing the same tibial cut plane and the same femoral prosthesis flexion-extension angle, the analysis device 302 can use the performance metric to identify and select the implant alignment option having predicted gap(s) 116 that match the reference value(s). In other embodiments, the analysis device 302 may apply a threshold filter to select a set of implant alignment options for which the performance metric is below a threshold.

Due to the culling process of block 1206, at least one degree of freedom of the selected set of implant alignment options is constrained relative to the initial set of degrees of freedom reflected in the initial set of implant alignment options. In many cases, block 1206 will result in multiple degrees of freedom being constrained (e.g., having fewer possible values) relative to the initial set of degrees of freedom. In some cases, one or more degrees of freedom present in the initial set of implant alignment options may even be fixed in the selected set of implant alignment options (i.e., all implant alignments in the selected set may share the same position with respect to that type of translation and/or rotation). It will be appreciated that these constraints on one or more degrees of freedom for the prosthetic components result in the selected set of implant alignment options consisting of fewer possible implant alignments than the initial set of implant alignment options.

After block 1206, the method 1202 proceeds to block 406 in which the analysis device 302 determines the predicted performance of the knee prosthesis for each implant alignment of the selected set of implant alignment options identified in block 1206. Block 406 of method 1202 may include any or all of the features described above for block 406 of method 400 (FIG. 6), including any or all features of the process 500 (FIG. 7). For example, the analysis device 302 may determine performance of the knee prosthesis using a performance mathematical model 336 and may determine predicted ligament elongations for each implant alignment in the selected set of implant alignment options. In contrast to method 400, however, in the illustrative embodiment of method 1202, block 406 is only performed on the smaller, selected set of implant alignment options and not on the larger, initial set of implant alignment options utilized for predicting flexion-extension gaps in block 1204. This can reduce the computational load on the analysis device 302 since generating each performance prediction in block 406 is frequently more computationally intensive than generating each predicted flexion-extension gap in block 1204.

After determining the predicted performance of the knee prosthesis for each implant alignment of the selected set of implant alignment options in block 406, the method 1202 advances to block 408 in which the analysis device 302 identifies one or more recommended implant alignments for the selected orthopaedic knee prosthesis 100. Block 408 of method 1202 may include any or all of the features described above for block 408 of method 400 (FIG. 6). For example, the analysis device 302 may evaluate and compare the target kinematics determined in block 404 of method 1202 with the performance predictions determined in block 406 of method 1202 (e.g., using a cost function).

After the analysis device 302 has selected the recommended implant alignment(s) of the orthopaedic knee prosthesis 100, the method 1202 advances to block 410 in which one or more of the recommended implant alignment(s) are incorporated into a patient-specific surgical plan 800. Block 410 of method 1202 may include any or all of the features described above for block 410 of method 400 (FIG. 6). For example, bock 410 may involve either preparing a new surgical plan 800 that includes the recommended implant alignment(s) or modifying portions of an existing surgical plan 800 that was previously developed for the patient. The surgical plan 800 may be displayed to surgeon electronically and/or in hard copy, either prior to surgery, during surgery, or both.

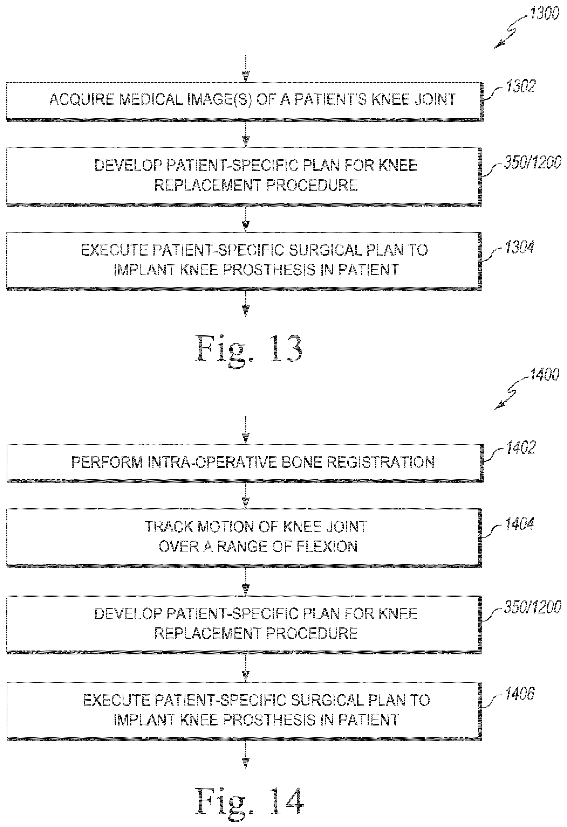

As noted above, the analysis device 302 is configured to prepare the patient-specific surgical plan based on medical images, intra-operatively identified anatomic features, or a combination of such images and features. Referring now to FIG. 13, a method 1300 shows one embodiment for developing a patient-specific surgical plan based on medical images and executing that plan. The method 1300 includes block 1302 in which the imaging device 304 is used to acquire the required medical images of the patient's anatomy. For example, the medical images in this embodiment are taken via CT scans but may be embodied as other types of two-dimensional medical images and/or three-dimensional medical images in other embodiments.

Next, the method 1300 implements either module 350 (see FIG. 6) or module 1200 (see FIG. 12). In block 402 of the implemented module 350/1200, the analysis device 302 may identify the relevant anatomical parameters based on manually annotated medical images received from the orthopaedic surgeon. In the illustrative embodiment, the anatomical parameters include the center and radius of the femoral medial condyle, the center and radius of the femoral lateral condyle, the posterior slope(s) of the tibial plateaus (medial and lateral), the varus-valgus angle of the tibia articular surface, the rotational angle to the tibial tuberosity, and the rotational angle to the fibular head. In other embodiments, the anatomical parameters may additionally or alternatively include any anatomical landmarks of the tibia center, the femur center, the femoral epicondyles (medial and lateral), and the malleoli, including the posterior cruciate ligament (PCL) attachment site and the most distal and posterior point(s) on the femoral medial and/or lateral condyle(s), by way of example. Various axes, such as the tibial sagittal axis, Whiteside's line, and the femoral and tibial mechanical axes, may also be determined from the medical images.

In some embodiments, the analysis device 302 may be configured to automatically and/or autonomously identify anatomical parameters on the patient's bony anatomy in the medical images. This autonomous identification may be in addition to or replace the manual annotations provided by the orthopaedic surgeon. For example, in some embodiments, the analysis device 302 may utilize a machine-learning algorithm (e.g., image processing module 332) to identify the anatomical landmarks and other anatomical parameters in the medical image(s). In such embodiments, the machine learning algorithm may undergo a training phase in which the machine learning algorithm is supplied with a training set of manually annotated medical images of sample patients. In this way, the machine-learning algorithm is trained to identify the corresponding anatomical parameters in new medical images such as the medical images of the present patient. After the anatomical parameters are determined, the analysis device 302 may perform the other activities of the implemented module 350/1200, described in greater detail above with reference to FIGS. 6, 7, and 12.

After development of the patient-specific surgical plan using module 350 or module 1200, the method 1300 advances to block 1304 in which the surgeon executes the patient-specific surgical plan 800 to implant the prosthetic components in the patient. To do so, the surgeon may use manual instruments to locate the resection planes 802 outlined in the plan 800 and place cutting guides and other surgical instruments in the desired positions to prepare the patient's bones to receive the prosthetic components. It should be appreciated that the surgeon may use customized, patient-specific instrumentation configured with features that align the instruments with the planned resection planes. The surgeon may also use augmented reality devices to display the surgical plan and to guide the placement of the manual instruments.

Referring now to FIG. 14, in another embodiment, the surgeon may follow a method 1400 to develop and execute a patient-specific plan utilizing the robotic surgical device 602. The method 1400 may begin with the surgeon performing initial surgical procedures on the patient's knee joint. For example, the orthopaedic surgeon may open the patient's knee joint envelope to allow access to the patient's boney anatomy. Additionally, in this embodiment, the orthopaedic surgeon may attach optical tracking arrays to each of the patient's femur and tibia of the corresponding knee joint to facilitate tracking of the patient's boney anatomy in three-dimensional space by the surgical device 602.

In block 1402, the surgeon performs a bone registration procedure. To do so, the orthopaedic surgeon may utilize the optical stylus 626 to identify particular anatomical parameters, which are recorded by the surgical device 602. In this embodiment, the anatomical parameters illustratively include the center and radius of the femoral medial condyle, the center and radius of the femoral lateral condyle, the posterior slope(s) of the tibial plateaus (medial and lateral), the varus-valgus angle of the tibia articular surface, the rotational angle to the tibial tuberosity, and the rotational angle to the fibular head. In other embodiments, the anatomical parameters may additionally or alternatively include any anatomical landmarks of the tibia center, the femur center, the femoral epicondyles (medial and lateral), and the malleoli, including the posterior cruciate ligament (PCL) attachment site and the most distal and posterior point(s) on the femoral medial and/or lateral condyle(s), by way of example. Various axes, such as the tibial sagittal axis, Whiteside's line, and the femoral and tibial mechanical axes, may also be determined from the bone registration procedure of block 1402.

In block 1404, the surgeon may move the patient's knee joint through a range of flexion and/or perform other movements of the patient's knee joint. The surgical device 602 tracks the patient's boney anatomy (i.e., the patient's femur and tibia) based on the attached optical tracking arrays and records the motion of the patient's boney anatomy. In this illustrative embodiment, the surgeon may use the bone distraction tool 626 to adjust the distance or gap between the patient's femur and tibia over the range of flexion. The tool 626 may be configured to generate distance or force data, which may be recorded for use in module 350.

The method 1400 advances to either module 350 or module 1200 in which the analysis device 302 (which may be incorporated into or separate from the surgical device 602) develops a patient-specific surgical plan based on the bone registration information and the tracking data recorded in blocks 1302, 1304. For example, when using either module 350 or module 1200, the analysis device 302 may determine the target kinematics of the patient's knee joint (block 404) based on the tracked movements and the anatomic landmarks. As described above, the target kinematics may include the varus-valgus angle, the internal-external rotation, the medial-lateral translation, the anterior-posterior translation, and the superior-inferior translation through a range of flexion. With these target kinematics, the analysis device 302 develops the patient-specific surgical plan incorporating the recommended implant alignment, as described in greater detail above with reference to FIGS. 6, 7, and 12.

In block 1406, the orthopaedic surgeon may perform the orthopaedic surgery to implant the selected orthopaedic knee prosthesis 100 based on the recommended knee prosthesis alignment. While doing so, the orthopaedic surgeon may further adjust the implant alignment and/or ligament elongations and, in response, the surgical device 602 updates the displayed alignment and ligament elongations based on such adjustments.

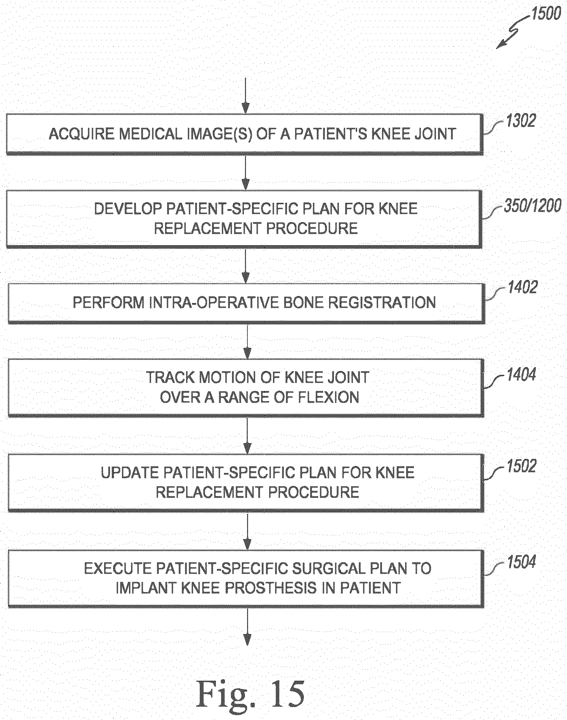

Referring now to FIG. 15, in some embodiments, the computer system 300 may be configured to perform a method 1500 to determine positioning of the knee prosthesis 100 based on medical images and intra-operatively generated information. As shown in FIG. 15, the method 1500 includes block 1302, module 350 or 1200, block 1402, and block 1404, each of which function as described above with reference to FIGS. 6, 7, 12, 13, and 14. The method 1500 also includes block 1502 in which the analysis device 302 updates the patient-specific surgical plan developed in module 350 or module 1200 (depending on the embodiment) based on the intra-operatively acquired data from blocks 1402 and 1404. The surgical device 602 may display both a pre-operative recommended implant alignment identified using the medical images and the intra-operative recommended implant alignment generated in block 1502.

In block 1502, the analysis device 302 may reconcile the pre-operative recommended implant alignment and the intra-operative recommended implant alignment using any suitable algorithm. For example, in some embodiments, the analysis device 302 may select one of the recommended implant alignments based on a particular criteria, average the two recommended implant alignments, and/or apply another mathematical operation to the recommended implant alignments to determine a final recommended implant alignment of the orthopaedic knee prosthesis 100.

Subsequently, the surgical device 602 is configured to display a visualization of the finalized, recommended knee prosthesis alignment and the associated ligament elongation(s) on the display 616. Additionally, in some embodiments, the orthopaedic surgeon may adjust the implant alignment and/or ligament elongations, and the surgical device 602 updates the displayed alignment and ligament elongations based on the surgeon's adjustments.

In block 1504, the orthopaedic surgeon may perform the orthopaedic surgery to implant the selected orthopaedic knee prosthesis 100 based on the finalized, recommended knee prosthesis alignment parameters. While doing so, the orthopaedic surgeon may further adjust the implant alignment and/or ligament elongations and, in response, the surgical device 602 updates the displayed alignment and ligament elongations based on such adjustments.

It should be appreciated that each of the methods described in reference to FIGS. 13-15 are in a simplified level of detail and that each of the methods 1300, 1400, and 1500 may include additional or alternative process steps in other embodiments. For example, each of the methods 1300, 1400, and 1500 may include additional initialization procedures, user interface interactions, coordinate systems construction steps, and/or other process steps and/or sub-flows.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the scope of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the scope of the present disclosure as defined by the appended claims.

## Claims

1. An orthopaedic surgical planning method (1202) for a knee joint of a patient, comprising:
identifying (402), by a computer system (302), anatomical parameters of the knee joint,
determining (404), by the computer system (302), target kinematics of the knee joint based on the patient's pre-implantation anatomy,
determining (1204), by the computer system (302), one or more predicted gaps (116) between a femoral prosthesis (102) and a tibial prosthesis (104) when implanted in the knee joint at each of a first set of implant alignment options, wherein the first set of implant alignment options reflect a first set of degrees of freedom for positioning of the femoral and tibial prostheses (102, 104) relative to bones (110, 112) of the knee joint,
determining (1206), by the computer system (302), a second set of implant alignment options from among the first set of implant alignment options based at least in part on the one or more predicted gaps (116), wherein the second set of implant alignment options reflect a second set of degrees of freedom for positioning of the femoral and tibial prostheses (102, 104) relative to the bones (110, 112) of the knee joint, and wherein at least one degree of freedom of the second set of degrees of freedom is constrained relative to the first set of degrees of freedom,
determining (406), by the computer system (302), predicted kinematics for each implant alignment of the second set of implant alignment options, wherein determining (406) the predicted kinematics includes the computer system (302) inputting the identified anatomical parameters into a mathematical model (336) representative of the kinematics of the femoral and tibial prostheses (102, 104) when implanted and operating (510) the mathematical model (336) to determine the predicted kinematics of the femoral and tibial prostheses (102, 104) for each implant alignment of the second set of implant alignment options,
identifying (408), by the computer system (302), at least one recommended implant alignment of the femoral and tibial prostheses (102, 104) based on the target kinematics and the predicted kinematics, and
incorporating (410), by the computer system (302), the at least one recommended implant alignment into a patient-specific surgical plan (800).

2. The method of claim 1, wherein multiple degrees of freedom of the second set of degrees of freedom are constrained relative to the first set of degrees of freedom.

3. The method of claim 1 or claim 2, wherein at least one degree of freedom of the first set of degrees of freedom is fixed for the second set of implant alignment options.

4. The method of any one of claims 1-3, wherein the computer system (302) determines (1204) the one or more predicted gaps (116) using the target kinematics and known geometries of the femoral and tibial prostheses (102, 104).

5. The method of any one of claims 1-4, wherein determining (1204) the one or more predicted gaps (116) comprises determining, for each implant alignment of the first set of implant alignment options, the predicted gap (116) between the femoral and tibial prostheses (102, 104) at each of a plurality of flexion-extension angles.

6. The method of any one of claims 1-5, wherein determining (1204) the one or more predicted gaps (116) comprises determining, for each implant alignment of the first set of implant alignment options, a medial-side predicted gap (116a) between the femoral and tibial prostheses (102, 104) and a lateral-side predicted gap (116b) between the femoral and tibial prostheses (102, 104).

7. The method of any one of claims 1-6, wherein determining (1206) the second set of implant alignment options from among the first set of implant alignment options comprises selecting implant alignments for which the one or more predicted gaps (116) are within a target range.

8. The method of any one of claims 1-7, wherein:
determining (404) the target kinematics of the knee joint includes determining a target ligament elongation of at least one ligament of the knee joint over a range of flexion,
operating (510) the mathematical model to determine the predicted kinematics of the femoral and tibial prostheses (102, 104) includes determining a predicted ligament elongation (934, 936) of the at least one ligament over the range of flexion, and
identifying (408) the at least one recommended implant alignment includes calculating a difference between the target ligament elongation and the predicted ligament elongation (934, 936) over the range of flexion for each implant alignment of the second set of implant alignment options and recommending the implant alignment corresponding to a least difference between the target ligament elongation and the predicted ligament elongation (934, 936) over the range of flexion.

9. The method of claim 8, wherein:
calculating the difference between the target ligament elongation and the predicted ligament elongation (934, 936) over the range of flexion includes calculating a sum of absolute differences between the target ligament elongation and the predicted ligament elongation (934, 936) in a number of poses throughout the range of flexion, and
recommending the implant alignment corresponding to the least difference includes recommending the implant alignment corresponding to the least sum of absolute differences.

10. The method of claim 8 or claim 9, wherein the at least one ligament is at least one of a medial collateral ligament, a lateral collateral ligament, a posterior cruciate ligament, an anterolateral ligament, and a posterior capsule of the knee joint of the patient.

11. The method of claim 10, wherein the at least one ligament comprises at least the medial collateral ligament, the lateral collateral ligament, and the posterior cruciate ligament.

12. The method of any one of claims 1-11, wherein identifying (402) anatomical parameters of the knee joint of the patient includes identifying anatomical parameters of the knee joint based on a set of medical images (330) of the patient's pre-operative anatomy.

13. The method of claim 12, further comprising acquiring the set of medical images (1302) through at least one of a CT scan, X-Ray imaging, and ultrasound imaging of the patient's pre-operative anatomy.

14. The method of any one of claims 1-13, wherein the mathematical model (336) is a linear response model configured to receive anatomic parameters, prostheses size, prostheses type, and implant alignment as inputs and to generate predicted kinematics of the femoral and tibial prostheses (102, 104) as an output.

15. The method of claim 14, wherein the linear response model is further configured to generate predicted mechanics of the femoral and tibial prostheses (102, 104) as an output, the mechanics including at least one of predicted contact forces between the femoral prosthesis (102) and the tibial prosthesis (104) and predicted contact forces between the femoral and tibial prostheses (102, 104) and the bones (110, 112) of the knee joint.

16. The method of any of claims 1-13, further comprising adding to the patient-specific surgical plan a number of planned resections of the patient's femur and at least one planned resection of the patient's tibia to prepare the patient's bones to receive the femoral and tibial prostheses at the recommended implant alignment.

17. An orthopaedic surgical planning system (300) comprising a computer system (302) configured to automatically perform a method (1202) according to any one of claims 1-16.

18. The system (300) of claim 17, further comprising at least one of:
a robotic system (602) including a cutting tool configured to resect one or more of the patient's bones (110, 112) according to the patient-specific surgical plan (800), or
a bone registration tool (626) for identifying, with the computer system (302), a number of landmarks on the patient's pre-implantation anatomy, wherein the anatomical parameters include the number of landmarks.

## Patentansprüche

1. Ein orthopädisches chirurgisches Planungsverfahren (1202) für ein Kniegelenk eines Patienten, das Folgendes beinhaltet:
Identifizieren (402), durch ein Computersystem (302), anatomischer Parameter des Kniegelenks,
Bestimmen (404), durch das Computersystem (302), einer Zielkinematik des Kniegelenks basierend auf der Vorimplantationsanatomie des Patienten,
Bestimmen (1204), durch das Computersystem (302), einer oder mehrerer vorhergesagter Lücken (116) zwischen einer Femurprothese (102) und einer Tibiaprothese (104), wenn sie in das Kniegelenk mit jeder aus einem ersten Satz von Implantatausrichtungsoptionen implantiert sind, wobei der erste Satz von Implantatausrichtungsoptionen einen ersten Satz von Freiheitsgraden zum Positionieren der Femur- und Tibiaprothese (102, 104) relativ zu Knochen (110, 112) des Kniegelenks widerspiegelt,
Bestimmen (1206), durch das Computersystem (302), eines zweiten Satzes von Implantatausrichtungsoptionen aus dem ersten Satz von Implantatausrichtungsoptionen basierend mindestens teilweise auf der einen oder den mehreren vorhergesagten Lücken (116), wobei der zweite Satz von Implantatausrichtungsoptionen einen zweiten Satz von Freiheitsgraden zum Positionieren der Femur- und Tibiaprothese (102, 104) relativ zu den Knochen (110, 112) des Kniegelenks widerspiegelt und wobei mindestens ein Freiheitsgrad des zweiten Satzes von Freiheitsgraden relativ zu dem ersten Satz von Freiheitsgraden eingeschränkt ist,
Bestimmen (406), durch das Computersystem (302), einer vorhergesagten Kinematik für jede Implantatausrichtung des zweiten Satzes von Implantatausrichtungsoptionen,
wobei das Bestimmen (406) der vorhergesagten Kinematik umfasst, dass das Computersystem (302) die identifizierten anatomischen Parameter in ein mathematisches Modell (336) eingibt, das die Kinematik der Femur- und Tibiaprothese (102, 104) darstellt, wenn sie implantiert sind, und das mathematische Modell (336) betreibt (510), um die vorhergesagte Kinematik der Femur- und Tibiaprothese (102, 104) für jede Implantatausrichtung des zweiten Satzes von Implantatausrichtungsoptionen zu bestimmen,
Identifizieren (408), durch das Computersystem (302), mindestens einer empfohlenen Implantatausrichtung der Femur- und Tibiaprothese (102, 104) basierend auf der Zielkinematik und der vorhergesagten Kinematik, und
Einbeziehen (410), durch das Computersystem (302), der mindestens einen empfohlenen Implantatausrichtung in einen patientenspezifischen chirurgischen Plan (800).

2. Verfahren gemäß Anspruch 1, wobei mehrere Freiheitsgrade des zweiten Satzes von Freiheitsgraden relativ zu dem ersten Satz von Freiheitsgraden eingeschränkt sind.

3. Verfahren gemäß Anspruch 1 oder Anspruch 2, wobei mindestens ein Freiheitsgrad des ersten Satzes von Freiheitsgraden für den zweiten Satz von Implantatausrichtungsoptionen festgelegt ist.

4. Verfahren gemäß einem der Ansprüche 1-3, wobei das Computersystem (302) die eine oder die mehreren vorhergesagten Lücken (116) unter Verwendung der Zielkinematik und bekannter Geometrien der Femur- und Tibiaprothese (102, 104) bestimmt (1204).

5. Verfahren gemäß einem der Ansprüche 1-4, wobei das Bestimmen (1204) der einen oder der mehreren vorhergesagten Lücken (116) das Bestimmen, für jede Implantatausrichtung des ersten Satzes von Implantatausrichtungsoptionen, der vorhergesagten Lücke (116) zwischen der Femur- und Tibiaprothese (102, 104) bei jedem aus einer Vielzahl von Flexions-Extensionswinkeln beinhaltet.

6. Verfahren gemäß einem der Ansprüche 1-5, wobei das Bestimmen (1204) der einen oder der mehreren vorhergesagten Lücken (116) das Bestimmen, für jede Implantatausrichtung des ersten Satzes von Implantatausrichtungsoptionen, einer medialseitigen vorhergesagten Lücke (116a) zwischen der Femur- und Tibiaprothese (102, 104) und einer lateralseitigen vorhergesagten Lücke (116b) zwischen der Femur-und Tibiaprothese (102, 104) beinhaltet.

7. Verfahren gemäß einem der Ansprüche 1-6, wobei das Bestimmen (1206) des zweiten Satzes von Implantatausrichtungsoptionen aus dem ersten Satz von Implantatausrichtungsoptionen das Auswählen von Implantatausrichtungen beinhaltet, für die die eine oder die mehreren vorhergesagten Lücken (116) innerhalb eines Zielbereichs liegen.

8. Verfahren gemäß einem der Ansprüche 1-7, wobei:
das Bestimmen (404) der Zielkinematik des Kniegelenks das Bestimmen einer Zielligamentverlängerung von mindestens einem Ligament des Kniegelenks über einen Beugungsbereich umfasst,
das Betreiben (510) des mathematischen Modells, um die vorhergesagte Kinematik der Femur- und Tibiaprothese (102, 104) zu bestimmen, das Bestimmen einer vorhergesagten Ligamentverlängerung (934, 936) des mindestens einen Ligaments über den Beugungsbereich umfasst, und
das Identifizieren (408) der mindestens einen empfohlenen Implantatausrichtung das Berechnen einer Differenz zwischen der Zielligamentverlängerung und der vorhergesagten Ligamentverlängerung (934, 936) über den Beugungsbereich für jede Implantatausrichtung des zweiten Satzes von Implantatausrichtungsoptionen und das Empfehlen der Implantatausrichtung, die einer geringsten Differenz zwischen der Zielligamentverlängerung und der vorhergesagten Ligamentverlängerung (934, 936) über den Beugungsbereich entspricht, umfasst.

9. Verfahren gemäß Anspruch 8, wobei:
das Berechnen der Differenz zwischen der Zielligamentverlängerung und der vorhergesagten Ligamentverlängerung (934, 936) über den Beugungsbereich das Berechnen einer Summe von absoluten Differenzen zwischen der Zielligamentverlängerung und der vorhergesagten Ligamentverlängerung (934, 936) in einer Anzahl von Posen über den gesamten Beugungsbereich umfasst, und
das Empfehlen der Implantatausrichtung, die der geringsten Differenz entspricht, das Empfehlen der Implantatausrichtung, die der geringsten Summe von absoluten Differenzen entspricht, umfasst.

10. Verfahren gemäß Anspruch 8 oder Anspruch 9, wobei das mindestens eine Ligament mindestens eines von einem medialen Kollateralligament, einem lateralen Kollateralligament, einem posterioren Kreuzband, einem anterolateralen Ligament und einer posterioren Kapsel des Kniegelenks des Patienten ist.

11. Verfahren gemäß Anspruch 10, wobei das mindestens eine Ligament mindestens das mediale Kollateralligament, das laterale Kollateralligament und das posteriore Kreuzband beinhaltet.

12. Verfahren gemäß einem der Ansprüche 1-11, wobei das Identifizieren (402) anatomischer Parameter des Kniegelenks des Patienten das Identifizieren anatomischer Parameter des Kniegelenks basierend auf einem Satz von medizinischen Bildern (330) der Voroperationsanatomie des Patienten umfasst.

13. Verfahren gemäß Anspruch 12, das ferner das Erfassen des Satzes von medizinischen Bildern (1302) durch mindestens eines von einem CT-Scan, Röntgenbildgebung und Ultraschallbildgebung der Voroperationsanatomie des Patienten beinhaltet.

14. Verfahren gemäß einem der Ansprüche 1-13, wobei das mathematische Modell (336) ein lineares Reaktionsmodell ist, das konfiguriert ist, um anatomische Parameter, Prothesengröße, Prothesentyp und Implantatausrichtung als Eingaben zu empfangen und um eine vorhergesagte Kinematik der Femur- und Tibiaprothese (102, 104) als Ausgabe zu erzeugen.

15. Verfahren gemäß Anspruch 14, wobei das lineare Reaktionsmodell ferner konfiguriert ist, um eine vorhergesagte Mechanik der Femur- und Tibiaprothese (102, 104) als Ausgabe zu erzeugen, wobei die Mechanik mindestens eines von vorhergesagten Kontaktkräften zwischen der Femurprothese (102) und der Tibiaprothese (104) und vorhergesagten Kontaktkräften zwischen der Femur- und Tibiaprothese (102, 104) und den Knochen (110, 112) des Kniegelenks umfasst.

16. Verfahren gemäß einem der Ansprüche 1-13, das ferner das Hinzufügen einer Anzahl von geplanten Resektionen des Femurs des Patienten und mindestens einer geplanten Resektion der Tibia des Patienten zu dem patientenspezifischen chirurgischen Plan beinhaltet, um die Knochen des Patienten vorzubereiten, um die Femur- und Tibiaprothese an der empfohlenen Implantatausrichtung zu empfangen.

17. Ein orthopädisches chirurgisches Planungssystem (300), das ein Computersystem (302) beinhaltet, das konfiguriert ist, um automatisch ein Verfahren (1202) gemäß einem der Ansprüche 1-16 durchzuführen.

18. System (300) gemäß Anspruch 17, das ferner mindestens eines der Folgenden beinhaltet:
ein Robotersystem (602), das ein Schneidwerkzeug umfasst, das konfiguriert ist, um einen oder mehrere der Knochen (110, 112) des Patienten gemäß dem patientenspezifischen chirurgischen Plan (800) zu resezieren, oder
ein Knochenregistrierungswerkzeug (626) zum Identifizieren, mit dem Computersystem (302), einer Anzahl von Orientierungspunkten auf der Vorimplantationsanatomie des Patienten, wobei die anatomischen Parameter die Anzahl von Orientierungspunkten umfassen.

## Revendications

1. Un procédé de planification chirurgicale orthopédique (1202) pour une articulation de genou d'un patient, comprenant :
l'identification (402), par un système informatique (302), de paramètres anatomiques de l'articulation de genou,
la détermination (404), par le système informatique (302), d'une cinématique cible de l'articulation de genou sur la base de l'anatomie de pré-implantation du patient,
la détermination (1204), par le système informatique (302), d'un ou de plusieurs écartements prédits (116) entre une prothèse fémorale (102) et une prothèse tibiale (104) lorsqu'elles sont implantées dans l'articulation de genou à chacune d'un premier ensemble d'options d'alignement d'implant, dans lequel le premier ensemble d'options d'alignement d'implant reflète un premier ensemble de degrés de liberté pour le positionnement des prothèses fémorale et tibiale (102, 104) par rapport à des os (110, 112) de l'articulation de genou,
la détermination (1206), par le système informatique (302), d'un deuxième ensemble d'options d'alignement d'implant parmi le premier ensemble d'options d'alignement d'implant sur la base au moins en partie des un ou plusieurs écartements prédits (116),
dans lequel le deuxième ensemble d'options d'alignement d'implant reflète un deuxième ensemble de degrés de liberté pour le positionnement des prothèses fémorale et tibiale (102, 104) par rapport aux os (110, 112) de l'articulation de genou, et
dans lequel au moins un degré de liberté du deuxième ensemble de degrés de liberté est contraint par rapport au premier ensemble de degrés de liberté,
la détermination (406), par le système informatique (302), d'une cinématique prédite pour chaque alignement d'implant du deuxième ensemble d'options d'alignement d'implant, dans lequel la détermination (406) de la cinématique prédite inclut le fait que le système informatique (302) entre les paramètres anatomiques identifiés dans un modèle mathématique (336) représentatif de la cinématique des prothèses fémorale et
tibiale (102, 104) lorsqu'elles sont implantées et exploite (510) le modèle mathématique (336) pour déterminer la cinématique prédite des prothèses fémorale et tibiale (102, 104) pour chaque alignement d'implant du deuxième ensemble d'options d'alignement d'implant,
l'identification (408), par le système informatique (302), d'au moins un alignement d'implant recommandé des prothèses fémorale et tibiale (102, 104) sur la base de la cinématique cible et de la cinématique prédite, et
l'incorporation (410), par le système informatique (302), de l'au moins un alignement d'implant recommandé dans un plan chirurgical spécifique au patient (800).

2. Le procédé de la revendication 1, dans lequel de multiples degrés de liberté du deuxième ensemble de degrés de liberté sont contraints par rapport au premier ensemble de degrés de liberté.

3. Le procédé de la revendication 1 ou de la revendication 2, dans lequel au moins un degré de liberté du premier ensemble de degrés de liberté est fixe pour le deuxième ensemble d'options d'alignement d'implant.

4. Le procédé de l'une quelconque des revendications 1 à 3, dans lequel le système informatique (302) détermine (1204) les un ou plusieurs écartements prédits (116) à l'aide de la cinématique cible et de géométries connues des prothèses fémorale et tibiale (102, 104).

5. Le procédé de l'une quelconque des revendications 1 à 4, dans lequel la détermination (1204) des un ou plusieurs écartements prédits (116) comprend la détermination, pour chaque alignement d'implant du premier ensemble d'options d'alignement d'implant, de l'écartement prédit (116) entre les prothèses fémorale et tibiale (102, 104) à chacun d'une pluralité d'angles de flexion-extension.

6. Le procédé de l'une quelconque des revendications 1 à 5, dans lequel la détermination (1204) des un ou plusieurs écartements prédits (116) comprend la détermination, pour chaque alignement d'implant du premier ensemble d'options d'alignement d'implant, d'un écartement prédit côté médial (116a) entre les prothèses fémorale et tibiale (102, 104) et d'un écartement prédit côté latéral (116b) entre les prothèses fémorale et tibiale (102, 104).

7. Le procédé de l'une quelconque des revendications 1 à 6, dans lequel la détermination (1206) du deuxième ensemble d'options d'alignement d'implant parmi le premier ensemble d'options d'alignement d'implant comprend la sélection d'alignements d'implant pour lesquels les un ou plusieurs écartements prédits (116) sont dans une plage cible.

8. Le procédé de l'une quelconque des revendications 1 à 7, dans lequel :
la détermination (404) de la cinématique cible de l'articulation de genou inclut la détermination d'un allongement de ligament cible d'au moins un ligament de l'articulation de genou sur une plage de flexion,
l'exploitation (510) du modèle mathématique pour déterminer la cinématique prédite des prothèses fémorale et tibiale (102, 104) inclut la détermination d'un allongement de ligament prédit (934, 936) de l'au moins un ligament sur la plage de flexion, et
l'identification (408) de l'au moins un alignement d'implant recommandé inclut le calcul d'une différence entre l'allongement de ligament cible et l'allongement de ligament prédit (934, 936) sur la plage de flexion pour chaque alignement d'implant du deuxième ensemble d'options d'alignement d'implant et la recommandation de l'alignement d'implant correspondant à une différence la plus faible entre l'allongement de ligament cible et l'allongement de ligament prédit (934, 936) sur la plage de flexion.

9. Le procédé de la revendication 8, dans lequel :
le calcul de la différence entre l'allongement de ligament cible et l'allongement de ligament prédit (934, 936) sur la plage de flexion inclut le calcul d'une somme de différences absolues entre l'allongement de ligament cible et l'allongement de ligament prédit (934, 936) dans un nombre de poses sur toute la plage de flexion, et
la recommandation de l'alignement d'implant correspondant à la différence la plus faible inclut la recommandation de l'alignement d'implant correspondant à la somme la plus faible de différences absolues.

10. Le procédé de la revendication 8 ou de la revendication 9, dans lequel l'au moins un ligament est au moins un élément parmi un ligament collatéral médial, un ligament collatéral latéral, un ligament croisé postérieur, un ligament antérolatéral, et une capsule postérieure de l'articulation de genou du patient.

11. Le procédé de la revendication 10, dans lequel l'au moins un ligament comprend au moins le ligament collatéral médial, le ligament collatéral latéral, et le ligament croisé postérieur.

12. Le procédé de l'une quelconque des revendications 1 à 11, dans lequel l'identification (402) de paramètres anatomiques de l'articulation de genou du patient inclut l'identification de paramètres anatomiques de l'articulation de genou sur la base d'un ensemble d'images médicales (330) de l'anatomie pré-opératoire du patient.

13. Le procédé de la revendication 12, comprenant en outre l'acquisition de l'ensemble d'images médicales (1302) par l'intermédiaire d'au moins une technique parmi une tomodensitométrie, une imagerie par rayons X, et une imagerie par ultrasons de l'anatomie pré-opératoire du patient.

14. Le procédé de l'une quelconque des revendications 1 à 13, dans lequel le modèle mathématique (336) est un modèle de réponse linéaire configuré pour recevoir des paramètres anatomiques, une taille de prothèse, un type de prothèse, et un alignement d'implant en tant qu'entrées et pour générer une cinématique prédite des prothèses fémorale et tibiale (102, 104) en tant que sortie.

15. Le procédé de la revendication 14, dans lequel le modèle de réponse linéaire est en outre configuré pour générer une mécanique prédite des prothèses fémorale et tibiale (102, 104) en tant que sortie, la mécanique incluant au moins un élément parmi des forces de contact prédites entre la prothèse fémorale (102) et la prothèse tibiale (104) et des forces de contact prédites entre les prothèses fémorale et tibiale (102, 104) et les os (110, 112) de l'articulation de genou.

16. Le procédé de n'importe lesquelles des revendications 1 à 13, comprenant en outre l'ajout au plan chirurgical spécifique au patient d'un nombre de résections planifiées du fémur du patient et d'au moins une résection planifiée du tibia du patient pour préparer les os du patient à recevoir les prothèses fémorale et tibiale à l'alignement d'implant recommandé.

17. Un système de planification chirurgicale orthopédique (300) comprenant un système informatique (302) configuré pour effectuer automatiquement un procédé (1202) selon l'une quelconque des revendications 1 à 16.

18. Le système (300) de la revendication 17, comprenant en outre au moins un élément parmi :
un système robotique (602) incluant un outil de coupe configuré pour réséquer un ou
plusieurs des os du patient (110, 112) selon le plan chirurgical spécifique au patient (800), ou
un outil de repérage osseux (626) pour identifier, avec le système informatique (302),
un nombre de points de repère sur l'anatomie de pré-implantation du patient, dans lequel les paramètres anatomiques incluent le nombre de points de repère.
